# EUROPEAN PATENT APPLICATION

(11) **EP 4 640 827 A1**
(43) Date of publication of application: **29.10.2025**
(21) Application number: 24172176.0
(22) Date of filing: 24.04.2024
(51) Int. Cl.: C12N 9/02, C12N 15/52, C12N 15/62

(54) **RECOMBINANT TET3 ENZYMES**

(71) Applicant: Ludwig-Maximilians-Universität München, 80539 München (DE)
(72) Inventor: The designation of the inventor has not yet been filed
(74) Representative: Weickmann & Weickmann PartmbB

(57) **Abstract**

The present invention refers to a recombinant Ten Eleven Translocation 3 (TET3) enzyme, a nucleic acid molecule encoding the TET3-enzyme, a vector comprising the nucleic acid molecule and a host cell transformed or transfected with the vector, the use of the recombinant TET3-enzyme for sequencing of a nucleic acid substrate, a method for sequencing a nucleic acid substrate and a reagent for sequencing a nucleic acid substrate.

## Description

### Field of the invention

The present invention refers to a recombinant Ten Eleven Translocation 3 (TET3) enzyme, a nucleic acid molecule encoding the TET3-enzyme, a vector comprising the nucleic acid molecule and a host cell transformed or transfected with the vector, the use of the recombinant TET3-enzyme for sequencing of a nucleic acid substrate, a method for sequencing a nucleic acid substrate and a reagent for sequencing a nucleic acid substrate.

### Background of the invention

DNA methylation is one of the important modifications of genomes in eukaryotic cells and plays a key role in mammalian cell development, differentiation, genomic imprinting and regulation of gene expression. It has important biological significance and is associated with human diseases (including tumor diseases) that are closely related to the occurrence and development processes. Therefore, it is of great value and significance to discover and establish genomic DNA modification profiles related to disease processes.

The presence of the 5^{th} nucleoside 5-methyldeoxycytidine (mdC) either in promoter regions or in the gene body influences the transcriptional state of the corresponding gene [1]. The presence of the methylated nucleobase mdC in promoter regions typically silences the gene, while unmethylated promoters stand for more active transcription. The identification of mdC within genes allows consequently to characterize the transcriptional state of the gene of interest, which is for example important in order to characterize and identify tumor cells [2-3], in which oncogenes are typically wrongly switched on and tumor suppressor genes are aberrantly switched off. Sequencing of mdC with as little input material as possible is consequently highly desired in order to establish a new area of early tumor diagnostic, called liquid biopsy [4].

Since sequencing of mdC positions in the genome is of paramount importance, especially for early cancer diagnostics in order to determine incorrect expression of genes, many attempts have been made to provide an accurate sequencing of mdC.

Until today, mdC sequencing is predominantly performed with bisulfite. Treatment of genomic DNA with bisulfite at 64°C converts all non-methylated cytidines into uracil, while mdC remains intact. Following PCR and sequencing and by comparing the obtained reads with a reference genome therefore allows to determine the position of the mdCs in the genome. The main problem associated with this method, however, is that a large amount of the genomic input DNA does not survive the harsh bisulfite treatment conditions due to extensive DNA fragmentation. Thus, this method cannot be applied for limited input samples.

This limitation may be overcome by extensive PCR-based amplification of the non-degraded DNA. For example, C. Liu et al. describe a mdC-specific whole-genome amplification system for simultaneous DNA amplification and methylation in a one-pot, primer-free reaction [5]. This method is specifically directed to samples having limited input materials in order to increase DNA amount prior to sequencing. After amplification, the amplified products are subjected to standard bisulfite sequencing.

Another caveat is that the handling of the bisulfite sequencing protocol is cumbersome and error prone. Milder methods that are currently being developed like, EM-seq, make use of the deaminating enzyme APOBEC3A (A3A), which deaminates dC to dU [6]. Deamination of all dC bases to dU, however, creates the problem that it decreases the genome complexity from a four letter code to a now only three nucleobase code (dA, dG and dU), which, plus the base mdC, makes the mathematical sequence assembly challenging.

An alternative approach for sequencing mdC is based on 3^{rd} generation sequencing where one directly reads out sequences without the need for a PCR step. Today, all 3^{rd} generation single molecule sequencing tools such as Nanopore or SMRT sequencing indeed allow a direct read-out of mdC [7]. These methods, however, are still at an early stage and the obtained signal differences between the sequencing signals obtained for dC and mdC are often very small. This further requires cumbersome deconvolution of the data with substantial bioinformatics [8-9].

CN 111220760 A describes a method for determining the concentration of mdC in plasma DNA. The method adopts a liquid chromatography-mass spectrometry system for determination, and comprises the following steps: taking a DNA sample extracted from plasma; adding a quantity of formic acid and carrying out reacting in a closed reaction bottle; carrying out cooling to room temperature, carrying out volatilizing in vacuum, adding methanol for dissolving, and carrying out centrifuging at a high speed; and taking supernatant for chromatographic column separation, and carrying out detecting by using a mass spectrum detector. However, the method needs heating the DNA samples to 70°C. Further, a strong acidic environment is needed, which finally hydrolyses mdC groups.

Mild epigenetic mdC sequencing methods, which would circumvent cytidine deamination are consequently highly desirable in order to provide a non-invasive, accurate, time, cost and resource saving early tumor diagnosis.

Current limitations for mdC sequencing could be overcome by 5-carboxycytidine (cadC) sequencing. cadC exhibits in contrast to dC an additional carboxy group, which is negatively charged under neural pH-conditions. This may provide a large signal difference between the neutral mdC and the negatively charged cadC. T. A. Clark et al. describe a method of detecting cadC instead of mdC by SMRT sequencing [10]. However, a proper oxidizing enzyme for effective and quantitative mdC to cadC oxidation is presently not available. Further, robust data analysis algorithms have hindered an implementation of this approach so far. Thus, this technology will benefit from a more proper type of oxidizing enzyme with high selectivity, stability, affinity, and turnover rates regarding the mdC substrate.

### Summary of the Invention

The present disclosure provides a truncated but robust, recombinant Ten Eleven Translocation 3 (TET3) enzyme comprising:
a) a first TET3 domain comprising an amino acid sequence having amino acids 696-1048 of Uniprot No. A0A5K1VVP6 (SEQ ID NO: 1) or an amino acid sequence having an identity of at least 85% over the whole length thereof;
b) a second TET3 domain comprising an amino acid sequence having amino acids 1509-1599 of Uniprot No. A0A5K1VVP6 (SEQ ID NO: 1) or an amino acid sequence having an identity of at least 85% over the whole length thereof, and
c) a connecting domain located between domain (a) and domain (b) comprising an amino acid sequence of at least 5 amino acids which is heterologous to a TET3 enzyme.

A further aspect of the present invention relates to a nucleic acid molecule encoding a TET3-enzyme as described above, optionally linked to an expression control sequence.

Still a further aspect of the invention relates to a vector comprising a nucleic acid molecule as described above.

Still a further aspect of the invention relates to a host cell transformed or transfected with a vector as described above.

Still a further aspect of the invention relates to a use of a recombinant TET3-enzyme as described above for the sequencing of a nucleic acid substrate, particularly for the sequencing of a nucleic acid substrate comprising mdC and/or 5-methylcytdine (mC) nucleotides.

Still a further aspect of the invention relates to a method for sequencing a nucleic acid substrate comprising mdC and/or mC nucleotides, wherein the method comprises the steps:
(A) contacting the nucleic acid substrate with a recombinant TET3-enzyme as described above under conditions, where mdC and/or mC nucleotides in the nucleic acid substrate are oxidized, e.g., to 5-hydroxymethyl-2'-deoxycytidine (hmdC), 5-formyl-2'-deoxycytidine (fdC), cadC, 5-hydroxymethylcytidine (hmC), 5-formylcytidine (fC), and/or 5-carboxycytidine (caC),
(B) optionally isolating the oxidized nucleic acid substrate, and
(C) determining the amount and/or position of mdC and/or mC nucleotides in the nucleic acid substrate.

A further aspect of the present invention relates to a reagent for sequencing a nucleic acid substrate comprising mdC and/or mC nucleotides comprising:
(A) a recombinant TET3-enzyme as described above, and
(B) an oxidizing agent.

### Embodiments of the invention

In the following, specific embodiments of the invention are disclosed as follows:
1. A recombinant Ten Eleven Translocation 3 (TET3) enzyme, comprising:
   a) a first TET3 domain comprising an amino acid sequence having amino acids 696-1048 of Uniprot No. A0A5K1VVP6 (SEQ ID NO: 1) or an amino acid sequence having an identity of at least 85% over the whole length thereof;
   b) a second TET3 domain comprising an amino acid sequence having amino acids 1509-1599 of Uniprot No. A0A5K1VVP6 (SEQ ID NO: 1) or an amino acid sequence having an identity of at least 85% over the whole length thereof, and
   c) a connecting domain located between domain (a) and domain (b) comprising an amino acid sequence of at least 5 amino acids which is heterologous to a TET3 enzyme.
2. The recombinant TET3-enzyme of embodiment 1, wherein domain (a) further comprises C-terminal extension of up to 5 amino acids, particularly with an amino acid sequence selected from IQKEK (SEQ ID NO: 5), LQKEK (SEQ ID NO. 6) or any partial sequence thereof.
3. The recombinant TET3-enzyme of embodiment 1 or 2, wherein domain (b) further comprises C-terminal extension of up to 5 amino acids, particularly with an amino acid sequence selected from AARLG (SEQ ID NO: 7) or any partial sequence thereof.
4. The recombinant TET3-enzyme of any one of embodiments 1-3, wherein domain
   (a) comprises:
      (i) an amino acid sequence having amino acids 696-1048 of Uniprot No. A0A5K1VVP6 (SEQ ID NO: 1) or an amino acid sequence having an identity of at least 90% over the whole length thereof;
      (ii) an amino acid sequence having amino acids 831-1183 of Uniprot No. Q8BG87 (SEQ ID NO: 2) or an amino acid sequence having an identity of at least 90% over the whole length thereof;
      (iii)an amino acid sequence having amino acids 824-1175 of Uniprot No. 043151 (SEQ ID NO: 3) or an amino acid sequence having an identity of at least 90% over the whole length thereof; or
      (iv)an amino acid sequence having amino acids 953-1304 of Uniprot No. A0JP82 (SEQ ID NO: 4) or an amino acid sequence having an identity of at least 90% over the whole length thereof.
5. The recombinant TET3-enzyme of any one of embodiments 1-4, wherein domain (b) comprises:
   (i) an amino acid sequence having amino acids 1509-1599 of Uniprot No. A0A5K1VVP6 (SEQ ID NO: 1) or an amino acid sequence having an identity of at least 90% over the whole length thereof;
   (ii) an amino acid sequence having amino acids 1644-1734 of Uniprot No. Q8BG87 (SEQ ID NO: 2) or an amino acid sequence having an identity of at least 90% over the whole length thereof;
   (iii) an amino acid sequence having amino acids 1635-1726 of Uniprot No. 043151 (SEQ ID NO: 3) or an amino acid sequence having an identity of at least 90% over the whole length thereof; or
   (iv)an amino acid sequence having amino acids 1742-1833 of Uniprot No. A0JP82 (SEQ ID NO: 4) or an amino acid sequence having an identity of at least 90% over the whole length thereof.
6. The recombinant TET3-enzyme of any one of embodiments 1-5, wherein the connecting domain has a length of 5-100, preferably 5-30, more preferably 10-20, and most preferably about 15 amino acids.
7. The recombinant TET3-enzyme of any one of embodiments 1-6, wherein the connecting domain consists of amino acids selected from G, S, A, T, E, N, D, and/or K.
8. The recombinant TET3-enzyme according to any one of embodiments 1-7, wherein the connecting domain has the amino acid sequence

   [(Gₙ)Xₘ]ᵣ,

   wherein X is in each occurrence independently selected from S, E or D,
   n is 1-5, particularly 2-4,
   m is 0-5, particularly 1-3,
   r is 1-5, particularly 2-4.
9. The recombinant TET3-enzyme of any one of embodiments 1-8, wherein the connecting domain has the amino acid sequence of SEQ ID NO: 8: GGGGSGGGGSGGGGS or SEQ ID NO: 9: GGGSGGGGSGGGGE or SEQ ID NO: 10 GGGGSGGGGSGGGGD.
10.The recombinant TET3-enzyme of any one of embodiments 1-9, which does not contain amino acid portions of a TET3 enzyme having a length of 20 amino acids or more, of 10 amino acids or more, and particularly of 6 amino acids or more outside domain (a) and domain (b).
11. The recombinant TET3-enzyme of any one of embodiments 1-10,
   which does not contain amino acid portions of SEQ ID NO: 1 having a length of 20 amino acids or more, of 10 amino acids or more, and particularly of 6 amino acids or more outside amino acids 696-1048 and amino acids 1509-1599 of Uniprot No. A0A5K1WP6; and/or
   which does not contain amino acid portions of SEQ ID NO: 2 having a length of 20 amino acids or more, of 10 amino acids or more, and particularly of 6 amino acids or more outside amino acids 831-1183 and amino acids 1644-1734 of Uniprot No. Q8BG87; and/or
   which does not contain amino acid portions of SEQ ID NO: 3 having a length of 20 amino acids or more of 10 amino acids or more, and particularly of 6 amino acids or more outside amino acids 824-1175 and amino acids 1635-1726 of Uniprot No. 043151; and/or
   which does not contain amino acid portions of SEQ ID NO: 4 having a length of 20 amino acids or more, of 10 amino acids or more, and particularly of 6 amino acids or more outside amino acids 953-1304 and amino acids 1742-1833 of Uniprot No. A0JP82.
12. The recombinant TET3-enzyme of any one of embodiments 1-11, which does not contain:
   (1) the amino acids corresponding to positions 1-695 of Uniprot No. A0A5K1VVP6 or an amino acid sequence having an identity of at least 90% over the whole length thereof;
   (2) the amino acids corresponding to positions 1049-1509 of Uniprot No. A0A5K1VVP6 or an amino acid sequence having an identity of at least 90% over the whole length thereof; and/or
   (3) the amino acids corresponding to positions 1600-1668 of Uniprot No. A0A5K1VVP6 or an amino acid sequence having an identity of at least 90% over the whole length thereof.
13.The recombinant TET3-enzyme of any one of embodiments 1-12, which does not contain:
   (1) the amino acids corresponding to positions 1-830 of Uniprot No. Q8BG87 or an amino acid sequence having an identity of at least 90% over the whole length thereof;
   (2) the amino acids corresponding to positions 1184-1643 of Uniprot No. Q8BG87 or an amino acid sequence having an identity of at least 90% over the whole length thereof; and/or
   (3) the amino acids corresponding to positions 1735-1803 of Uniprot No. Q8BG87 or an amino acid sequence having an identity of at least 90% over the whole length thereof.
14.The recombinant TET3-enzyme of any one of embodiments 1-13, which does not contain:
   (1) the amino acids corresponding to positions 1-823 of Uniprot No. 043151 or an amino acid sequence having an identity of at least 90% over the whole length thereof;
   (2) the amino acids corresponding to positions 1176-1634 of Uniprot No. 043151 or an amino acid sequence having an identity of at least 90% over the whole length thereof; and/or
   (3) the amino acids corresponding to positions 1727-1795 of Uniprot No. 043151 or an amino acid sequence having an identity of at least 90% over the whole length thereof.
15. The recombinant TET3-enzyme of any one of embodiments 1-14, which does not contain:
   (1) the amino acids corresponding to positions 1-952 of Uniprot No. A0JP82 or an amino acid sequence having an identity of at least 90% over the whole length thereof;
   (2) the amino acids corresponding to positions 1305-1741 of Uniprot No. A0JP82 or an amino acid sequence having an identity of at least 90% over the whole length thereof; and/or
   (3) the amino acids corresponding to positions 1834-1901 of Uniprot No. A0JP82 or an amino acid sequence having an identity of at least 90% over the whole length thereof.
16.The recombinant TET3-enzyme of any one of embodiments 1-15, wherein
   amino acid T at position 940 of Uniprot No. A0A5K1VVP6 is substituted with another amino acid, particularly with an amino acid selected from A, G, K and/or V; or
   amino acid T at position 1075 of Uniprot No. Q8BG87 is substituted with another amino acid, particularly with an amino acid selected from A, G, K, and/or V; or
   amino acid T at position 1067 of Uniprot No. 043151 is substituted with another amino acid, particularly with an amino acid selected from A, G, K and/or V; or
   amino acid T at position 1196 of Uniprot No. A0JP82 is substituted with another amino acid, particularly with an amino acid selected from A, G, K and/or V.
17.The recombinant TET3-enzyme of any one of embodiments 1-16, wherein
   amino acid Y at position 1567 of Uniprot No. A0A5K1VVP6 is substituted with another amino acid, particularly with an amino acid selected from F, M and/or W; or
   amino acid Y at position 1702 of Uniprot No. Q8BG87 is substituted with another amino acid, particularly with an amino acid selected from F, M and/or W; or
   amino acid Y at position 1694 of Uniprot No. 043151 is substituted with another amino acid, particularly with an amino acid selected from F, M and/or W; or
   amino acid Y at position 1901 of Uniprot No. A0JP82 is substituted with another amino acid, particularly with an amino acid selected from F, M and/or W.
18.The recombinant TET3-enzyme of any one of embodiments 1-17, which catalyzes oxidation of 5-methyl-2'-deoxycytidine (mdC) to 5-hydroxymethyl-2'-deoxycytidine (hmdC), 5-formyl-2'-deoxycytidine (fdC) and/or 5-carboxy-2'-deoxycytidine (cadC), and which optionally catalyzes oxidation of 5-methylcytdine (mC) to 5-hydroxymethylcytidine (hmC), 5-formylcytidine (fC), and/or 5-carboxycytidine (caC).
19.The recombinant TET3-enzyme of any one of embodiments 1-18, which catalyzes oxidation of mdC selectively to cadC.
20.A nucleic acid molecule encoding the TET3-enzyme of any one of embodiments 1-19, optionally linked to an expression control sequence.
21. The nucleic acid molecule of embodiment 20 having a codon-optimized sequence for expression in bacteria.
22.Avector comprising a nucleic acid molecule of any one of any one of embodiments 20-21.
23. A host cell transformed or transfected with a vector of embodiment 22.
24. The host cell of embodiment 23, which is a prokaryotic or eukaryotic cell, preferably a prokaryotic cell.
25. The host cell of embodiment 23 or 24, which is an E. *coli* cell.
26.Use of a recombinant TET3-enzyme of any one of embodiments 1-19 for the sequencing of a nucleic acid substrate, particularly for the sequencing of a nucleic acid substrate comprising mdC and/or mC nucleotides.
27. The use of embodiment 26, wherein mdC nucleotides are oxidized to hmdC, fdC and/or cadC, and/or mC nucleotides are oxidized to hmC, fC, and/or caC.
28.The use of any one of embodiments 26-27, wherein the sequencing procedure does not involve bisulfite addition.
29. The use of any one of embodiments 26-28, wherein the sequencing procedure is a single molecule-sequencing procedure.
30. The use of any one of embodiments 26-29 in diagnostics, preferably (early) cancer diagnostics, such as cancer screening.
31. The use of any one of embodiments 26-30 in biological assays, such as oxygenase assays, enzyme kinetic assays, drug screening assays, and/or drug selectivity profiling.
32.A method for sequencing a nucleic acid substrate comprising mdC and/or mC nucleotides, wherein the method comprises the steps:
   (A) contacting the nucleic acid substrate with a recombinant TET3-enzyme of any one of embodiments 1-19 under conditions, where mdC and/or mC nucleotides in the nucleic acid substrate are oxidized, e.g. to hmdC, fdC, cadC, hmC, fC, and/or caC
   (B) optionally isolating the oxidized nucleic acid substrate, and
   (C) determining the amount and/or position of mdC and/or mC nucleotides in the nucleic acid substrate.
33. The method of embodiment 32, wherein the oxidation in step (A) is performed with an oxidizing agent, particularly a hypervalent iodine reagent such as IBX, nitroxyl radical-generating agent such as TEMPO, MnO₂, chromate, or any combination thereof.
34.The method of any one of embodiments 32-33, which is a SMRT-sequencing method.
35.The method of any one of embodiments 32-34, wherein the determining step (C) comprises detecting oxidized nucleotides by chromatography, e.g., a HPLC-based chromatography, optionally in combination with MS.
36. The method of any one of embodiments 32-35, wherein the oxidation in step (A) takes place under conditions, wherein mdC and/or mC nucleotides are selectively oxidized to cadC and/or caC nucleotides, particularly in an amount of at least 90%, preferably at least 95%, more preferably at least 99%, even more preferably at least 99.9% based on the total amount of oxidized mdC and/or mC nucleotides.
37. The method of embodiment 36, wherein the oxidation in step (A) is performed at an ion strength corresponding to 50-120 mM sodium chloride, preferably corresponding to 60-100 mM sodium chloride.
38. The method of any one of embodiments 32-35, wherein the oxidation in step (A) takes place under conditions, wherein mdC and/or mC nucleotides are selectively oxidized to 5-hmdC, 5-hmC, 5-fdC and/or 5-fC nucleotides, particularly in an amount of at least 50%, preferably at least 70%, more preferably at least 90% based on the total amount of oxidized mdC and/or mC nucleotides.
39. The method of embodiment 38, wherein the oxidation in step (A) is performed at an ion strength corresponding to 130-160 mM sodium chloride, preferably corresponding to 140-150 mM sodium chloride.
40. The method of any one of embodiments 32-35, wherein the oxidation in step (A) takes place under conditions, wherein mdC and/or mC are selectively oxidized to 5-fdC and/or 5-fC, particularly to at least 50%, preferably at least 70%, more preferably more than 90% based on the total amount of oxidized mdC and/or mC nucleotides.
41. The method of embodiment 40, wherein the oxidation in step (A) is performed at an ion strength corresponding to 180-250 mM sodium chloride, preferably corresponding to 190-220 mM sodium chloride, even more preferably corresponding to 200-210 mM.
42.A reagent for sequencing a nucleic acid substrate comprising mdC and/or mC nucleotides comprising:
   (A) a recombinant TET3-enzyme of any one of embodiments 1-19 and
   (B) an oxidizing agent.
43.The reagent of embodiment 42, wherein the oxidizing agent is selected from the group consisting of a hypervalent iodine reagent such as IBX, nitroxyl radical-generating agent such as TEMPO, MnO₂, chromate, or any combination thereof.

### Detailed description

TET enzymes are alpha-ketoglutarate dependent Fe²⁺ dioxygenases that catalyze dioxygenase-mediated oxidation of mdC to 5-hydroxymethyl-2'-deoxycytidine (hmdC), 5-formyl-2'-deoxycytidine (fdC) and/or 5-carboxy-2'-deoxycytidine (cadC). So far, those oxidations were performed with difficult to overexpress TET1- and TET2- derived enzymes [6, 12-15]. Further, the known enzymes were not able to quantitatively oxidize the mdC substrate with high selectivity and turnover rates.

Surprisingly, a recombinant TET3-enzyme has been found, which can be efficiently produced by a cost, time and energy saving process. Moreover, the recombinant TET3-enzyme of the invention surprisingly provides highly selective and quantitative oxidation capability towards a mdC and/or mC containing nucleic acid substrate.

"TET3-enzyme" in the sense of the present invention refers to any vertebrate TET 3 enzyme, e.g., to a mammalian, amphibious or reptile TET3-enzyme, in particular human, mouse, or frog TET3-enzyme. TET3 plays a key role in epigenetic chromatin reprogramming during embryonic development and is implicated in demethylation in many biological processes such as zygote formation, embryogenesis, axon regeneration, and synaptic transmission.

In the human adult brain, the most prevalent TET3-enzyme is TET3, having the amino acid sequence of SEQ ID NO: 3 (Uniprot No. 043151). In mice, four isoforms exist, the longest isoform, having the amino acid sequence of SEQ ID NO: 2 (Uniprot No. Q8BG87) and three shorter isoforms, having the amino acid sequence of SEQ ID NO: 1 (Uniprot No. A0A5K1WP6), Uniprot No. Q8BG87-2 and Uniprot No. Q8BG87-4. In frogs, in particular in Xenopus tropicalis, the TET3 enzyme of the amino acid sequence SEQ ID NO: 4 (Uniprot No. A0JP82) is present.

Thus, a first aspect of the present invention is directed to a TET3-enzyme, comprising:
a) a first TET3 domain comprising an amino acid sequence having amino acids 696-1048 of Uniprot No. A0A5K1VVP6 (SEQ ID NO: 1) or an amino acid sequence having an identity of at least 85%, at least 90%, at least 95% or at least 98% over the whole length thereof;
b) a second TET3 domain comprising an amino acid sequence having amino acids 1509-1599 of Uniprot No. A0A5K1VVP6 (SEQ ID NO: 1) or an amino acid sequence having an identity of at least 85%, at least 90%, at least 95% or at least 98% over the whole length thereof, and
c) a connecting domain located between domain (a) and domain (b) comprising an amino acid sequence of at least 5 amino acids which is heterologous to a TET3 enzyme.

The TET3 enzyme of the invention comprises a first TET3 domain (a) and a second TET3 domain (b) linked by a heterologous connecting domain. The first and second TET3 domain comprise the indicated portions of the amino acid sequence of a TET3 enzyme as described above or an amino acid sequence having an identity of at least 85%, at least 90%, at least 95% or at least 98% to the mouse TET3 amino acid sequence Uniprot No. A0A5K1VVP6 (SEQ ID NO: 1) over the whole length thereof.

"Percent (%) amino acid sequence identity" with respect to a peptide or polypeptide sequence is defined as the percentage of amino acid residues in a candidate sequence that are identical with the amino acid residues in the specific peptide or polypeptide sequence, after aligning the sequences and introducing gaps, if necessary, to achieve the maximum percent sequence identity. Alignment for purposes of determining percent amino acid sequence identity can be achieved in various ways that are within the skill in the art, for instance using publicly available computer software such as BLAST.

In certain embodiments, domain (a) further comprises a C-terminal extension of up to 5 amino acids, particularly an amino acid sequence selected from IQKEK (SEQ ID NO. 5), LQKEK (SEQ ID NO. 6) or any partial sequence thereof. In certain embodiments, domain (b) further comprises a C-terminal extension of up to 5 amino acids, particularly an amino acid sequence selected from AARLG (SEQ ID NO. 7) or any partial sequence thereof.

In certain embodiments, domain (a) comprises an amino acid sequence having amino acids 696-1048 of Uniprot No. A0A5K1VVP6 (SEQ ID NO: 1) or an amino acid sequence having an identity of at least 90%, 95%, 98% or 99% over the whole length thereof.

In certain embodiments, domain (a) comprises an amino acid sequence having amino acids 831-1183 of Uniprot No. Q8BG87 (SEQ ID NO: 2) or an amino acid sequence having an identity of at least at least 90%, 95%, 98% or 99% over the whole length thereof,

In certain embodiments, domain (a) comprises an amino acid sequence having amino acids 824-1175 of Uniprot No. 043151 (SEQ ID NO: 3) or an amino acid sequence having an identity of at least 90%, 95%, 98% or 99% over the whole length thereof.

In certain embodiments, domain (a) comprises an amino acid sequence having amino acids 953-1304 of Uniprot No. A0JP82 (SEQ ID NO: 4) or an amino acid sequence having an identity of at least 90%, 95%, 98% or 99% over the whole length thereof.

In certain embodiments, domain (b) comprises an amino acid sequence having amino acids 1509-1599 of Uniprot No. A0A5K1VVP6 (SEQ ID NO: 1) or an amino acid sequence having an identity of at least 90%, 95%, 98% or 99% over the whole length thereof.

In certain embodiments, domain (b) comprises an amino acid sequence having amino acids 1644-1734 of Uniprot No. Q8BG87 (SEQ ID NO: 2) or an amino acid sequence having an identity of at least 90%, 95%, 98% or 99% over the whole length thereof.

In certain embodiments, domain (b) comprises an amino acid sequence having amino acids 1635-1726 of Uniprot No. 043151 (SEQ ID NO: 3) or an amino acid sequence having an identity of at least 90%, 95%, 98% or 99% over the whole length thereof.

In certain embodiments, domain (b) comprises an amino acid sequence having amino acids 1742-1833 of Uniprot No. A0JP82 (SEQ ID NO: 4) or an amino acid sequence having an identity of at least 90%, 95%, 98% or 99% over the whole length thereof.

The connecting domain connects domain (a) with domain (b). Preferably, the connecting domain has a length of 5-100, more preferably 5-30, even more preferably 10-20, and most preferably about 15 amino acids. In certain embodiments, the connecting domain consists of amino acids selected from G, S, A, T, E, N, D, and/or K.

Preferably, the connecting domain has the amino acid sequence

[(Gn)Xm]r,

wherein X is in each occurrence independently selected from S, E or D,
n is 1-5, particularly 2-4,
m is 0-5, particularly 1-3,
r is 1-5, particularly 2-4.

In a preferred embodiment, the connecting domain has the amino acid sequence of SEQ ID NO: 8: -GGGGSGGGGSGGGGS-. In a further preferred embodiment, the connecting domain has the amino acid sequence of SEQ ID NO: 9: -GGGGSGGGGSGGGGE- or SEQ ID NO: 10: -GGGGSGGGGSGGGGD-.

The recombinant TET3-enzyme may further comprise a further N- and/or C-terminal peptide domain heterologous to a TET3 enzyme as herein described above. In certain embodiments, the N- and/or C-terminal peptide domain has a length of 5-100 amino acids, preferably 5-50 amino acids, even more preferably 10-30 amino acids. The peptide domain may be an affinity tag for purification, e.g., a Strep tag, a poly-Histidinetag (e.g., a Hiss-tag), a chitin binding protein, a maltose binding protein, an albumin-binding protein, a cellulose binding protein, a choline-binding domain, FLAG-tag, or glutathione-S-transferase (GST)-tag.

In particular embodiments, the N- and/or C-terminal peptide domain comprises a Strep-tag, more preferably a Strep-tag II, e.g., MASWSHPQFEK (SEQ ID NO: 11).

The recombinant TET3-enzyme may further comprise a protease cleavage site for removing the N- and/or C-terminal heterologous peptide domain as described above. In a preferred embodiment the protease cleavage site is a TEV protease cleavage site, e.g., SEQ ID NO: 12, which is susceptible to cleavage by a TEV protease (Tobacco Etch Virus nuclear-inclusion-a endopeptidase).

In another preferred embodiment, the protease cleavage site is a PreScission protease cleavage site, e.g., SEQ ID NO: 13, which is susceptible to cleavage by a PreScission protease, a genetically engineered fusion protein of human rhinovirus 3C protease and glutathione S transferase (GST) or a Hiss-tagged HRV 3C protease cleavage site. Preferably, the protease cleavage site is N-terminally fused to domain (a) of the recombinant TET3-enzyme, e.g., to amino acid 696 of Uniprot No. A0A5K1VVP6, to amino acid 831 of Uniprot No. Q8BG87, to amino acid 824 of Uniprot No. 043151 or to amino acid 953 of Uniprot No. A0JP82.

In certain embodiments, the recombinant TET3-enzyme does not contain amino acid portions of a TET3 enzyme having a length of 20 amino acids or more, of 10 amino acids or more, or of 6 amino acids or more outside domain (a) and domain (b).

In certain embodiments, the recombinant TET3-enzyme does not contain amino acid portions of SEQ ID NO: 1 having a length of 20 amino acids or more, of 10 amino acids or more, particularly of 6 amino acids or more outside amino acids 696-1048 and amino acids 1509-1599.

In certain embodiments, the recombinant TET3-enzyme does not contain amino acid portions of SEQ ID NO: 2 having a length of 20 amino acids or more, of 10 amino acids or more, particularly of 6 amino acids or more outside amino acids 831-1183 and amino acids 1644-1734.

In certain embodiments, the recombinant TET3-enzyme does not contain amino acid portions of SEQ ID NO: 3 having a length of 20 amino acids or more, of 10 amino acids or more, or of 6 amino acids or more outside amino acids 824-1175 and amino acids 1635-1726.

In certain embodiments, the recombinant TET3-enzyme does not contain amino acid portions of SEQ ID NO: 4 having a length of 10 amino acids or more, particularly of 6 amino acids or more outside amino acids 953-1304 and amino acids 1742-1833.

In particular embodiments, the recombinant TET3-enzyme comprises at least one amino acid substitution compared to a naturally occurring TET3-enzyme that alters enzyme specificity and/or activity.

In certain embodiments, the amino acid T corresponding to amino acid T at position 940 of Uniprot No. A0A5K1VVP6, amino acid T at position 1075 of Uniprot No. Q8BG87, amino acid T at position 1067 of Uniprot No. 043151, and amino acid T at position 1196 is substituted with another amino acid, particularly with an amino acid selected from A, G, K and/or V, more particularly with A.

In a further preferred embodiment, the amino acid Y corresponding to amino acid Y at position 1567 of Uniprot No. A0A5K1VVP6, amino acid Y at position 1702 of Uniprot No. Q8BG87, amino acid Y at position 1694 of Uniprot No. 043151 or amino acid Y at position 1901 of Uniprot No. A0JP82 is substituted with another amino acid, particularly with F or W.

In a specific embodiment, the recombinant TET3-enzyme comprises
a) a first TET3 domain comprising an amino acid sequence having amino acids 696-1048 of SEQ ID NO: 1 or an amino acid sequence having an identity of at least 90%, 95%, 98% or 99% over the whole length thereof;
b) a second TET3 domain comprising an amino acid sequence having amino acids 1509-1599 of SEQ ID NO: 1 or an amino acid sequence having an identity of at least at least 90%, 95%, 98% or 99% over the whole length thereof, and
c) a connecting domain located between domain (a) and domain (b) comprising an amino acid sequence of at least 5 amino acids which is heterologous to a TET3 enzyme,
wherein
amino acid T at position 940 is substituted with another amino acid, preferably with an amino acid selected from A, G, K and/or V, more preferably A; and/or
amino acid Y at position 1567 is substituted with another amino acid, preferably with an amino acid selected from F.

In a further specific embodiment, the recombinant TET3-enzyme comprises
a) an amino acid sequence having amino acids 831-1183 of SEQ ID NO: 2 or an amino acid sequence having an identity of at least 90%, 95%, 98% or 99% over the whole length thereof;
b) an amino acid sequence having amino acids 1644-1734 of SEQ ID NO: 2 or an amino acid sequence having an identity of at least 90%, 95%, 98% or 99% over the whole length thereof; and
c) a connecting domain located between domain (a) and domain (b) comprising an amino acid sequence of at least 5 amino acids which is heterologous to a TET3 enzyme,
wherein
amino acid T at position 1075 is substituted with another amino acid, preferably with an amino acid selected from A, G, K and/or V, more preferably A; and/or
amino acid Y at position 1702 is substituted with another amino acid, preferably with an amino acid selected from F or W.

In a further specific embodiment, the recombinant TET3-enzyme comprises
a) an amino acid sequence having amino acids 824-1175 of SEQ ID NO: 3 or an amino acid sequence having an identity of at least 90%, 95%, 98% or 99% over the whole length thereof;
b) an amino acid sequence having amino acids 1635-1726 SEQ ID NO: 3 or an amino acid sequence having an identity of at least 90%, 95%, 98% or 99% over the whole length thereof; and
c) a connecting domain located between domain (a) and domain (b) comprising an amino acid sequence of at least 5 amino acids which is heterologous to a TET3 enzyme,
wherein
amino acid T at position 1067 is substituted with another amino acid, preferably with an amino acid selected from A, G, K and/or V, more preferably A; and/or
amino acid Y at position 1694 is substituted with another amino acid, preferably with F or W.

In a further specific embodiment, the recombinant TET3-enzyme comprises
a) an amino acid sequence having amino acids 953-1304 of SEQ ID NO: 4 or an amino acid sequence having an identity of at least 90%, 95%, 98% or 99% over the whole length thereof;
b) an amino acid sequence having amino acids 1743-1833 of SEQ ID NO: 4 or an amino acid sequence having an identity of at least 90%, 95%, 98% or 99% over the whole length thereof; and
c) a connecting domain located between domain (a) and domain (b) comprising an amino acid sequence of at least 5 amino acids which is heterologous to a TET3 enzyme,
wherein
amino acid T at position 1196 is substituted with another amino acid, preferably with an amino acid selected from A, G, K and/or V, more preferably; and/or
amino acid Y at position 1901 of Uniprot No. A0JP82 is substituted with another amino acid, preferably with F or W.

In a preferred embodiment, the recombinant TET3-enzyme has a calculated molecular weight of at least 45 kDa, preferably at least 50 kDa, more preferably 50-55 kDa, most preferably about 52 kDa.

The recombinant TET3-enzyme catalyzes the oxidation of 5-methyl-2'-deoxycytidine (mdC). In certain embodiments, mdC is oxidized to 5-hydroxymethyl-2'-deoxycytidine (hmdC), 5-formyl-2'-deoxycytidine (fdC) and/or 5-carboxy-2'-deoxycytidine (cadC). Optionally, the recombinant TET3-enzyme catalyzes oxidation of 5-methylcytdine (mC). In certain embodiments, mC is oxidized to 5-hydroxymethylcytidine (hmC), 5-formylcytidine (fC), and/or 5-carboxycytidine (caC).

In certain embodiments, the TET3-enzyme catalyzes preferably oxidation of mdC selectively to cadC.

In a second aspect, the present invention is directed to a nucleic acid molecule encoding the recombinant TET3-enzyme, optionally in operative linkage to an expression control sequence.

"Expression control sequence" in the sense of the present invention refers to a regulatory sequence, i.e. a segment of a nucleic acid molecule which is capable of increasing or decreasing the expression of specific genes within a host cell, e.g., a prokaryotic or eukaryotic host cell. Suitable expression control sequences are well known in the art. For expression in a prokaryotic host cell such as E. coli, a T7 polymerase, lactose/IPTG, arabinose (P_{BAD}) and/or anhydrotetracycline responsive expression control sequence may be used.

Preferably, the nucleic acid molecule has a codon-optimized sequence for expression in bacteria. Codon optimization improves the translation efficiency of a target gene in order to maximize protein expression from the DNA sequence. Preferably, the following codon optimization strategy was used: Choice of preferentially most prevalent codons in E. coli, while avoiding repetitive sequence elements that might impact on vector stability.

In a third aspect, the present invention is directed to a vector, comprising the nucleic acid molecule of above.

"Vector" in the sense of the present invention refers to an extrachromosomal vehicle, e.g. a plasmid or virus designed for introduction of a nucleic acid molecule into a host cell encoding the recombinant TET3-enzyme and optionally allowing expression of the nucleic acid molecule. The vector may contain an expression control sequence as described above in operative linage to the nucleic acid molecule encoding the recombinant TET3-enzyme.

In a further aspect, the present invention is directed to a host cell, transformed or transfected with the vector of above. The host cell is preferably a prokaryotic or eukaryotic cell, more preferably a prokaryotic cell. In a preferred embodiment, the host cell is an E. coli cell or Bacillus cell, most preferably an E. coli cell. In a further preferred embodiment, the host cell is a yeast cell, e.g. Saccharomyces cerevisiae, or an animal cell, particularly an insect or a mammalian cell, e.g. a human or a hamster cell.

The recombinant TET3-enzyme may be recombinantly produced in a suitable host cell, e.g. in a prokaryotic or eukaryotic host cell. For this purpose, a vector comprising a nucleic acid molecule encoding the recombinant TET3-enzyme may be introduced into the host cell and the host cell is cultivated under conditions allowing expression of the recombinant TET3-enzyme.

In a further aspect, the present invention is directed to a use of the recombinant TET3-enzyme for the sequencing of a nucleic acid substrate, e.g., a DNA substrate or an RNA substrate, comprising methylated nucleotides such as mdC and/or mC nucleotides. The nucleic acid substrate may be a single-or double stranded nucleic acid molecule, e.g., a DNA or an RNA molecule. In certain embodiments, the nucleic acid substrate is genomic DNA which may be obtained from a subject, e.g., a human subject.

In a preferred embodiment, the recombinant TET3-enzyme is incubated with the nucleic acid substrate under conditions wherein mdC nucleotides in the nucleic acid substrate are oxidized to hmdC, fdC and/or cadC, and/or mC nucleotides are oxidized to hmC, fC, and/or caC. Preferably, the total amount of oxidized nucleotides in the substrate is least about 50%, at least about 70% least about 90% or least about 99%.

In a preferred embodiment, a sequencing procedure using the recombinant TET3-enzyme does not involve bisulfite addition.

In a preferred embodiment, the nucleotide sequence of the oxidized substrate is determined by single molecule sequencing, e.g., by single molecule real-time (SMRT) sequencing. SMRT sequencing is a parallelized single molecule DNA sequencing method, which may utilize a zero-mode waveguide (ZMW). A single DNA polymerase enzyme molecule is immobilized on a ZMW support and contacted with a single molecule of DNA as a template. The ZMW is a structure that creates an illuminated observation volume that is small enough to observe only a single nucleotide of DNA being incorporated by DNA polymerase. Each of the four DNA bases is attached to one of four different fluorescent dyes. When a nucleotide is incorporated by the DNA polymerase, the fluorescent tag is cleaved off and diffuses out of the observation area of the ZMW where its fluorescence is no longer observable. A detector detects the fluorescent signal of the nucleotide incorporation, and the base call is made according to the corresponding fluorescence of the dye.

In a particular aspect, the present invention is directed to a method for sequencing a nucleic acid substrate comprising mdC and/or mC nucleotides, wherein the method comprises the steps:
(A) contacting the nucleic acid substrate with a recombinant TET3-enzyme as described above under conditions, where mdC and/or mC nucleotides in the nucleic acid substrate are oxidized, e.g., oxidized to hmdC, fdC, cadC, hmC, fC, and/or caC,
(B) optionally isolating the oxidized nucleic acid substrate, and
(C)determining the location and/or amount of oxidized mdC and/or mC nucleotides in the oxidized nucleic acid substrate.

Preferably, the nucleic acid substrate comprises a 5-methylcytidine (mdC) nucleotide or an analogue thereof according to formula (I) 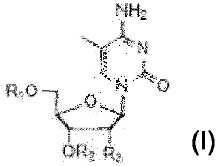 wherein
R1, and R2 are linkages to adjacent nucleotides; and
R3 is H or OH.

In a preferred embodiment, the recombinant TET3-enzyme oxidizes the nucleotide of formula (I) to hmC, hmdC and/or an analogue thereof according to formula (II) 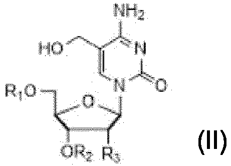 wherein
R1, and R2 are linkages to adjacent nucleotides; and
R3 is H or OH.
or
to fC, fdC and/or an analogue thereof according to formula (III), 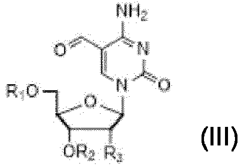 wherein
R1, and R2 are linkages to adjacent nucleotides; and
R3 is H or OH.
or
to caC, cadC or an analogue thereof according to formula (IV) 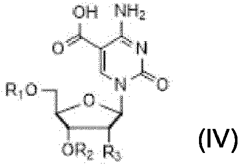 wherein
R1, and R2 are linkages to adjacent nucleotides; and
R3 is H or OH.
or any combination thereof.

The oxidation in step (A) is preferably performed in the presence of an oxidizing agent which is capable of oxidizing a nucleotide of formula (I) without substantially without disrupting the nucleic substrate. In certain embodiments, the oxidizing agent is selected from a hypervalent iodine reagent such as IBX, a nitroxyl radical generating compound such as 2,2,6,6-Tetramethyl-1-piperidinyloxy (TEMPO), MnO₂, chromate, or any combination thereof.

In certain embodiments, the oxidation in step (A) may take place under conditions, wherein mdC and/or mC nucleotides are selectively oxidized to cadC and/or caC nucleotides, particularly in an amount of at least 90%, preferably at least 95%, more preferably at least 99%, even more preferably at least 99.9% based on the total amount of oxidized mdC and/or mC nucleotides. Preferably, under these conditions the reaction takes place at an ion strength, i.e., an amount of monovalent cations and anions corresponding to 50-120 mM sodium chloride, preferably corresponding to 60-100 mM sodium chloride. The ion strength regulates presumably the on and off rates of the enzymes with the oligonucleotide target and for each oxidation a new α-ketoglutarate needs to be loaded into the enzyme.

Determining cadC nucleotides is particularly preferred in the context of SMRT sequencing by increasing the kinetic parameters IPD (inter-pulse duration) and PW (pulse width) and improving data evaluation. This may open the door for mild epigenetic cadC sequencing.

In certain embodiments, the oxidation in step (A) may take place under conditions, wherein mdC and/or mC nucleotides are selectively oxidized to 5-hmdC, 5-hmC, 5-fdC and/or 5-fC nucleotides, particularly in an amount of at least 50%, preferably at least 70%, more preferably at least 90% based on the total amount of oxidized mdC and/or mC nucleotides. Preferably, under these conditions the reaction takes place at an ion strength, i.e. an amount of monovalent cations and anions corresponding to 130-160 mM sodium chloride, preferably corresponding to 140-150 mM sodium chloride.

In certain embodiments, the oxidation in step (A) may take place under conditions, wherein mdC and/or mC are selectively oxidized to 5-fdC and/or 5-fC, particularly to at least 50%, preferably at least 70%, more preferably more than 90% based on the total amount of oxidized mdC and/or mC nucleotides. Preferably, under these conditions the reaction takes place at an ion strength, i.e. an amount of monovalent cations and anions corresponding to 180-250 mM sodium chloride, preferably corresponding to 190-220 mM sodium chloride, even more preferably corresponding to 200-210 mM sodium chloride.

In certain embodiments, the oxidation in step (A) may take place under conditions, wherein the undesired oxidative side products 8-oxodG and hmdU are generated in minor amounts. Thus, preferably, deoxyguanosine (dG) and/or deoxythymidine (dT) are oxidized to 8-oxodG and/or 5-hydroxymethyl-dU (hmdU), respectively, to at most 5%, more preferably to at most 2%, more preferably to at most 1%, more preferably to at most 0.05%, even more preferably to at most 0.01%, based on the total amount of oxidized nucleotides.

In certain embodiments, step (C) comprises determining the amount of oxidized nucleotides in the nucleic acid substrate without sequence analysis, e.g., by cleaving the nucleic acid substrate into individual nucleotides and detecting oxidized nucleotides. Preferably, the detection is performed quantitatively thereby allowing to determine the quantitative amount of methylated nucleotides in the nucleic acid substrate. The detection may be carried out by chromatography, e.g., HPLC-based chromatography, optionally in combination with MS. Preferably, quantitative triple quadruple mass spectrometry (UHPLC-QQQ-MS/MS) is used.

In certain embodiments, step (C) comprises determining the positions of oxidized nucleotides in the nucleic acid substrate involving a sequencing procedure, e.g., a SMRT sequencing method as described above.

Sequencing of mdC positions in the genome is of paramount importance for diagnostic applications such as early cancer diagnostics in order to determine incorrect expression of genes.

Thus, in a preferred embodiment, the recombinant TET3-enzyme is used in diagnostics, preferably (early) cancer diagnostics, such as cancer screening or the detection of the age status of a certain tissue or an organism. Further, the recombinant TET3-enzyme may be used in biological assays, drug screening assays, and/or drug selectivity profiling and/or assays in which the methylation state of a genetic element needs to be determined.

A further aspect of the present invention is directed to a reagent for sequencing a nucleic acid substrate comprising mdC and/or mC nucleotides comprising:
(A) a recombinant TET3-enzyme as described above, and
(B) an oxidizing agent.

Further, the invention is described in detail by the following figures and examples.

### Figure Legends

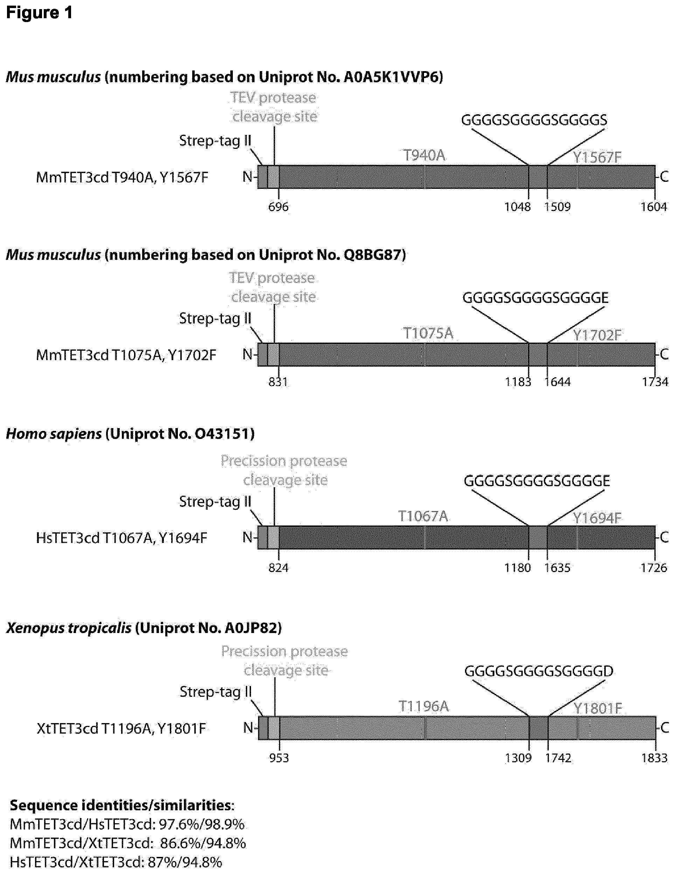

**Figure 1****: Summary of TET3 catalytic domains and constructs.**
   The Figure shows the preferred TET3-enzyme constructs according to the invention, based on Uniprot No. A0A5K1VVP6 (SEQ ID NO: 1) (Mus musculus, hereinafter "***hpTET3***"), Uniprot No. 043151 (SEQ ID NO: 3) (Homo sapiens) or Uniprot No. A0JP82 (SEQ ID NO: 4) (Xenopus tropicalis).
**Figure 2****: Purification of hpTET3.**

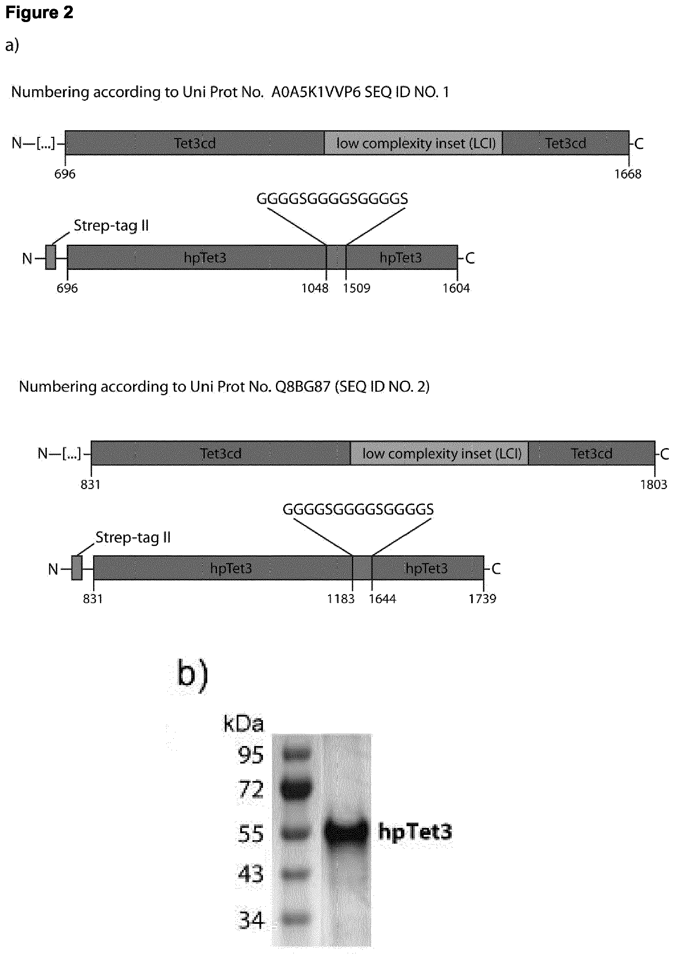

   Figure 2 a): Depiction of the domain structure of recombinant TET3-enzyme in comparison to the native isoform TET3 (on basis of SEQ ID NO: 1 (Uniprot No. A0A5K1VVP6) or SEQ ID NO: 2 (Q8BG87)). Figure 2 b): SDS-PAGE of the purified protein after two steps.

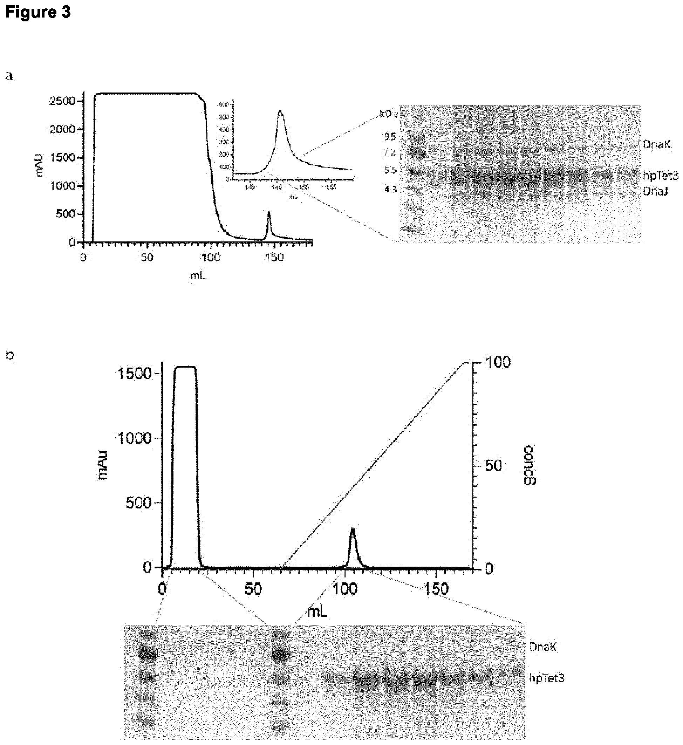

**Figure 3****: Two-step purification of hpTET3 enzyme expressed in E. coli.**
   N-terminally Strep(II)-tagged TET3-enzyme (hpTET3) was first purified by affinity chromatography on a StrepTrap XT resin. Contaminating DnaK and DnaJ chaperons were removed from hpTET3 by cation exchange chromatography using a HiTrap Heparin HP or HiTrap CaptoS column. Representative chromatograms and Coomassie-stained SDS-polyacrylamide gel electrophoresis analysis of the StrepTrap XT affinity chromatography elution fractions are shown in (A) and after Heparin chromatography in (B).

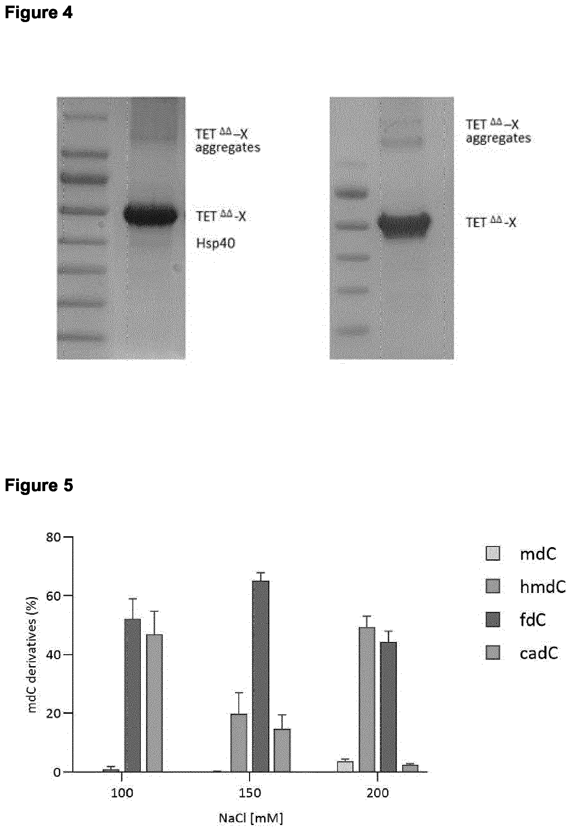

**Figure 4****: Purified recombinant hpTET3 enzyme.**
   Purified hpTET3 protein was loaded on a 4-15% gradient SDS-PAGE gel and stained with Coomassie Blue.
**Figure 5****: Recombinant TET3-enzyme protein activity.**
   The Figure show the influence of various salt concentrations on the distribution of the oxidation products hmdC, fdCd and cadC. At 150mM NaCl preferentially fdC is formed. At 200 mM NaCl a mixture of hmdC and fdC is generated.

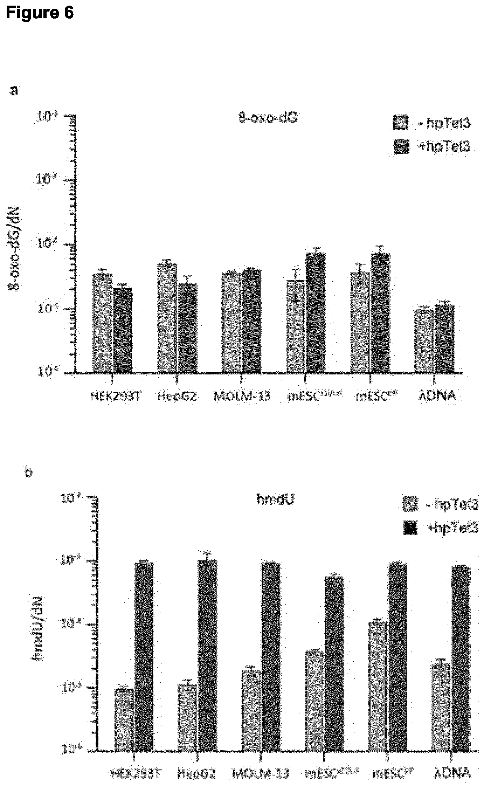

**Figure 6****: Oxidation of genomic DNA.**
   8-oxo-dG (a) and hmdU (b) levels per nucleosides (dN) of various genomic DNA before and after treatment with hpTET3 as quantified by UHPLC-QQQ-MS. Depicted are biological mean values with the respective ±SD.

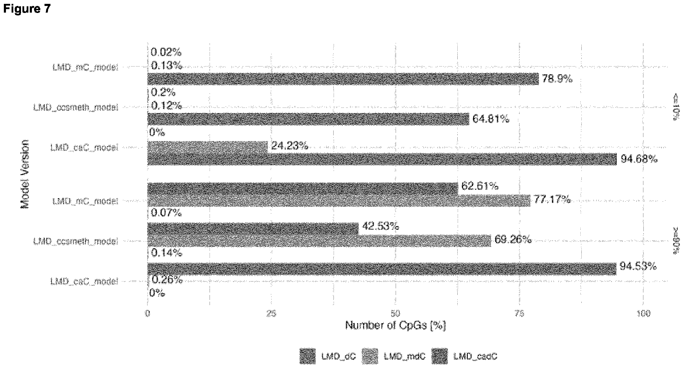

**Figure 7****: Modification Frequency of LMD-dC-, LMD-mdC- and LMD-cadC-CpGs.**
   The bars (x-axis) display the percentage of CpGs with a modification (methylation) frequency of 0 -10% (top plot, <=10%) and 90 - 100% (bottom plot, >= 90%) for the individual data sets, unmodified (LMD_dC), methylated (LMD-mdC) and carboxylated (LMD_cadC) obtained from applying the three trained deep-learning algorithm (y-axis).

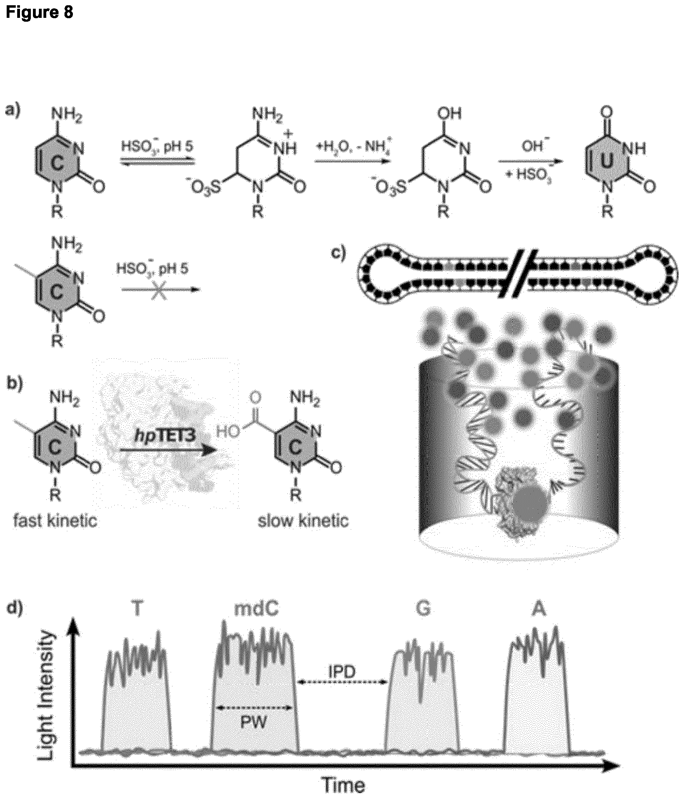

**Figure 8****: Comparison of bisulfite sequencing and SMRT sequencing.**
   8 a) Depiction of bisulfite sequencing method and 8 b) of the mdC to cadC oxidation chemistry as the basis for the sequencing method described in this publication. 8 c) Depiction of the SMRT sequencing concept with a circular sequencing DNA template bound to the polymerase and fluorescent labelled triphosphates. 8 d) Fluorescent signal during SMRT sequencing recorded by the Sequel Ile system. Y-axis shows the fluorescent intensity, x-axis the passing time. The kinetics or the nucleotide incorporation by the polymerase can be described by tow time constants. The inter pulse duration (IPD), the time between two light pulses (nucleotide incorporation) and the pulse width (PW) the time the polymerase needs to form the phosphodiester bond. The presence of non-canonical bases increase IPD and PW.

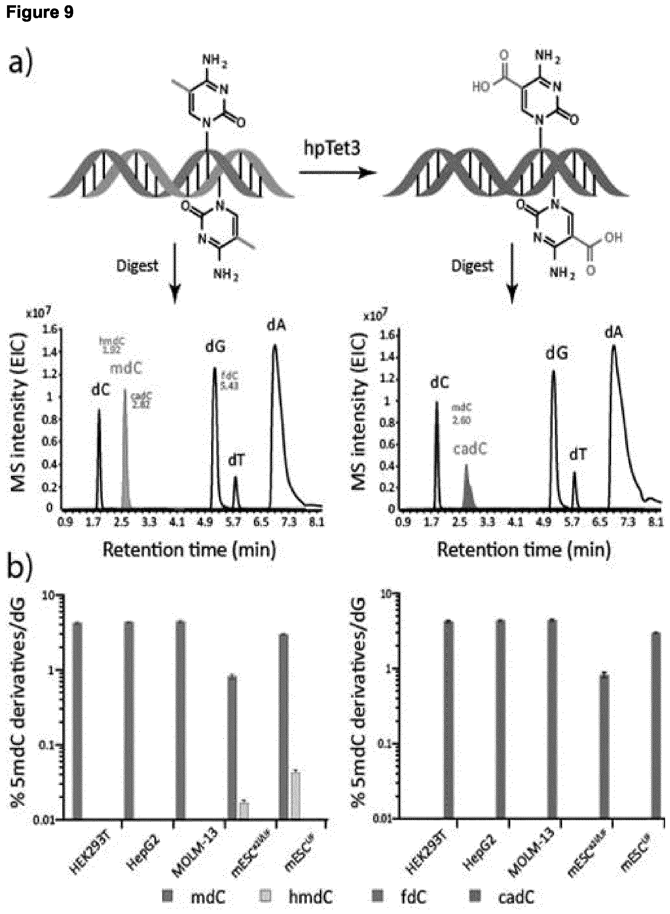

**Figure 9****: Oxidation of genomic DNA with hpTET3.**
   9 a) M.Sssl methylated λDNA(dam-,dcm-) was digested to single nucleosides, isotope standards were added (see Supplementary Table 1 for complete list) and the mixture analyzed by isotope dilution triple quadrupole mass spectrometry giving quantitative data for all nucleosides (left panel). The quantitative analysis of the nucleoside composition of genomic DNA was repeated after treatment of the genomic DNA with hpTET3 (right panel). 9 b) This analysis was performed on various human and mouse genomes with natural methylation levels.

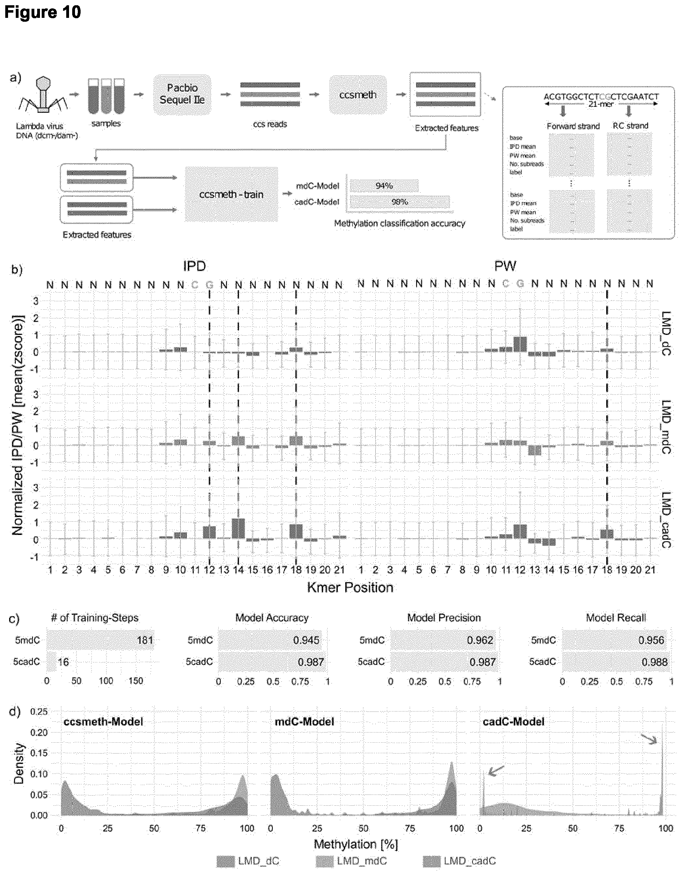

**Figure 10****: Application of hpTET3 for SMRT sequencing.**
   10 a) sequencing and model training workflow; dcm & dam negative DNA from lambda phage were sequenced using the Sequel Ile system, HiFi reads were aligned and IPD as well as PW values (features) extracted using ccsmeth; using ccsmeth we trained a 5mdC detecting model, as well as a cadC detecting model. 10 b) mean IPD & PW values (z-score normalized) extracted by ccsmeth across a 21kmer centred around a given for unmodified lambda DNA (LMD_dC, grey), the methylated lambda DNA (LMD_mdC, red) and the carboxylated lambda DNA (LMD_cadC, blue). 10 c) CpG model training parameters of the 5mdC and cadC model respectively. 10 d) density plot of the called unmodified cytosines (LMD_dC) and modified CpGs (LMD_5mdC or LMD_cadC).

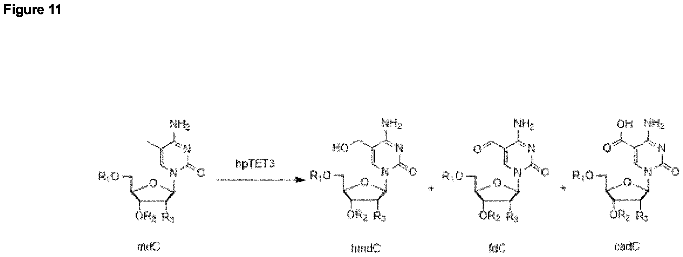

**Figure 11****: Depiction of the oxidation of mdC to hmdC, fdC and cadC.**
   While not-mutated hpTET3 generates exclusively and with high yield cadC (>99.9%), double-mutated hpTET3 (T940A and Y1567F) produces a mixture of hmdC and fdC with little cadC.

### Examples

### 1. Design of recombinant TET3-enzymes

Functional recombinant TET3-enzymes were designed which still comprise minimum regions necessary for catalysis (Δ means that this region of the sequence was deleted):
- Mus musculus TET3: amino acids 696 - 1604 of Uniprot No. A0A5K1VVP6 (SEQ ID NO: 1), Δ1049 - 1508.
- Mus musculus TET3: amino acids 831 - 1734 of Uniprot No. Q8BG87 (SEQ ID NO: 2), Δ1189-1641.
- Homo sapiens TET3: amino acids 824 - 1726 of Uniprot No. 043151 (SEQ ID NO: 3), Δ1181-1634.
- Xenopus tropicalis TET3: amino acids 953 - 1833 of Uniprot No. A0JP82 (SEQ ID NO: 4), Δ1310-1741.

### 2. Expression and purification of TET3-enzymes in E. coli

The for E. coli expression codon optimized synthetic sequence encoding a N-terminally Strep(II)-tagged truncated mouse TET3 protein (amino acids 696 - 1604 of Uniprot No. A0A5K1VVP6 (SEQ ID NO: 1) with residues 1049 - 1507 replaced by a 15-residue GS-linker GGGGSGGGGSGGGGS, SEQ ID NO: 8, named hereinafter "***hpTET3***") was designed, ordered from Life Technologies and sub-cloned into pET28a between the Ncol and Xhol restriction sites. In preparation of recombinant protein expression, BL21 (DE3) competent E. *coli* cells were transformed with Strep(II)-tagged hpTET3 plasmid and selected on Luria-Bertani (LB) plates containing kanamycin (25 µg/ml final concentration). A single colony was picked and cultured overnight at 37 °C in 50 mL of LB broth with appropriate antibiotic at 37 °C at 180 rpm in an Innova S44i incubator shaker (Eppendorf). For protein expression, bacteria from the overnight small-scale culture were diluted 1000-fold in LB medium with appropriate antibiotic and cells were grown at 37°C and 200 rpm until an optical density (OD₆₀₀) of 0.3 was reached. Then cultures were cooled down to 16°C and target protein expression was induced at an OD₆₀₀ between 0.5 and 0.6 by addition of isopropyl-β-D-thiogalactopyranoside to a final concentration of 0.5 mM. Furthermore, cells were grown in 2YT medium. Protein expression was carried out for 18 h. Subsequently, cells were harvested by centrifugation at 8000 rpm for 10 min at 4 °C, rinsed in cold PBS, then pelleted again at 8000 rpm. The pellet was flash-frozen in liquid nitrogen and stored at -80 °C until further use.

For purification, frozen cell pellets were thawed on ice, resuspended in ice-cold lysis buffer (50 mM HEPES pH 6.8, 500 mM NaCl, 10% glycerol, 0.5 mM TCEP, 10 µM ZnCl₂, 0.5 mg/ml lysozyme, EDTA-free protease inhibitor cocktail (Roche), 5 mM ATP and 5 mM MgCl₂) and incubated on ice for 30 min. To reduce viscosity from chromosomal DNA and RNA, lysates were treated with Benzonase. Cells were lysed using a high-pressure homogenizer (EmulsiFlex C5) and the cell lysate was cleared by centrifugation at 40,000 g for 45 min at 4 °C. Cleared lysate was loaded on a StrepTrap XT column (Cytiva 5 mL column) for affinity purification. After sample application, the column was washed with 8 column volumes (CV) of wash buffer (50 mM HEPES pH 6.8, 500 mM NaCl, 10% glycerol and 0.5 mM TCEP) and bound protein was eluted with 10 CV buffer containing 50 mM HEPES (pH 6.8), 100 mM NaCl, 10% glycerol, 0.5 mM TCEP and 50 mM biotin. Fractions containing hpTET3 were pooled and concentrated with an Amicon ultra centrifugal filter device (30 000 MWCO, 15 mL). Then, the eluate was diluted 1-fold with a buffer containing 50 mM HEPES pH 6.8, 100 mM NaCl, 10% glycerol, 10 mM ATP, 10 mM MgCl₂ and 0.5 mM TCEP and incubated for 30 min on ice. This procedure aims at the removal of contaminating tightly bound molecular chaperone Hsp70 (DnaK) - Hsp40 (DnaJ) - hpTET3 complexes. Subsequently, incorrectly folded hpTET3 aggregates were centrifuged for 30 min at 25,000 g at 4°C and the supernatant was applied to a HiTrap Heparin HP column (Cytiva, 5 ml) pre-equilibrated in binding buffer (50 mM HEPES pH 6.8, 100 mM NaCl, 10% glycerol (v/v), 0.5 mM TCEP). The column was washed with 20 CV of binding buffer and hpTET3 was eluted with a linear salt gradient ranging from 0.1 to 1.5 M NaCl (0%-100% elution buffer containing 50 mM HEPES pH 6.8, 1.5 M NaCl, 10% glycerol (v/v), 0.5 mM TCEP) over 20 CV. Alternatively, purification of the protein is possible with a CaptoS column without the ATP step.

hpTET3-containing fractions were buffer exchanged and concentrated to a final buffer containing 50 mM HEPES pH 6.8, 250 mM NaCl, 10% glycerol and 0.5 mM TCEP using Amicon centrifugal filters with a molecular weight cut-off of 30 kDa. Aliquots of pure hpTET3 were flash-frozen in liquid nitrogen and stored at -80°C. Protein purity was confirmed by Coomassie-stained SDS-PAGE. Typical yields were 2-3 mg per liter of E. *coli* culture.

All column chromatography steps were carried out at 4°C with pre-cooled buffers on a AKTA pure chromatography system and protein-containing fractions were kept on ice during whole purification procedure.

The purity of the protein according to analysis by SDS-PAGE is about 99% (**Figure 4****).**

### 3. Preparation of methylated lambda DNA

Lambda DNA (Oxford Nanopore: EXP-CTL001) was amplified by whole genome amplification (WGA) using the Direct WGA kit (Jena Bioscience, cat. no. PCR-382S) in order to produce unmodified DNA. The WGA was conducted according to Direct WGA kit's protocol - 1.0 ng lambda DNA was incubated with 12 µl reaction buffer, 1 mM dNTP Mix, 1 µl primer mix and 1 µl enzyme mix in a 20 µL reaction volume at 30°C for overnight (16 h) and followed by a heat inactivation for 5 min at 65°C.

Unmodified lambda DNA was sheared by Bioruptor^{®} Pico (Diagenode) (2 µg lambda DNA, 2 times of 2 cycles at 5"/90") to produce 1 kb size of fragmented DNA.

1 µg of unmethylated, sheared DNA was methylated in vitro using 4 U of M.Sssl enzyme (NEB, cat. no. M0226S) in the presence of 160 µM S-adenosylmethionine (SAM) (NEB, cat. no. B9003S) in the provided reaction buffer (1x) (10 mM Tris-HCl pH 7.9, 50 mM NaCl, 10 mM MgCl₂ and 1 mM DTT) (NEB, cat. no. B7002S) in a 50 µL reaction volume for 90 min at 37°C. To ensure complete CpG methylation, the reaction mixture was supplemented with additional 4 U of M.Sssl, 145 µM SAM and the reaction buffer (0.1x) in a 55 µL reaction volume. Then the reaction was incubated for an additional 90 min at 37°C followed by a heat inactivation for 20 min at 65°C. Methylated DNA was purified with 1.8x AMPure XP beads (Beckman Coulter, pro. no. A63882) according to the manufacturer's protocol. DNA methylation was confirmed by quantitative UHPLC-QQQ-MS/MS.

### 3. DNA oxidation with TET3-enzymes

All reactions were performed with 1 µg genomic DNA in a total volume of 50 µl at 37 °C for 1h at 500 rpm. DNA concentrations were quantified using 1x dsDNA-HS (High Sensitivity) Qubit Assay-Kit.

1 µg of human or mouse genomic DNA was incubated with 4 µM (10 µg) hpTET3 in buffer containing 50 mM HEPES, 50 mM NaCl, 1 mM α-ketoglutarate, 2 mM ascorbic acid, 1.2 mM ATP, 105 µM Fe(NH₄)₂(SO₄)₂ and 2.5 mM DTT at 37°C for 1 h. The pH of the reaction was 7.4 after addition of all buffer components. Alternatively, since 25.7% of total cytosines are methylated (mdC) in M.Sssl treated lambda DNA, compared to approximately 4.5% of total cytosines are mdC in human gDNA, 1 µg of M.Sssl treated lambda DNA was incubated with 11 µM (30 µg) recombinant TET3 at 37°C for 1 h with the same buffer condition. After that, 0.8 U of Proteinase K (NEB, cat. no. P8107S) and SDS to a final concentration of 0.05% w/v were added to the reaction mixture and incubated for 1h at 50 °C. Subsequently, oxidized DNA was purified with 1.8x AMPure XP beads (Beckman Coulter) according to the manufacturer's protocol. Oxidation efficiency was quantified by UHPLC-QQQ-MS/MS.

It is important to note that ascorbic acid and Fe(NH₄)₂(SO₄)₂ must be freshly prepared and Fe(II) should be dissolved in water and added immediately before the reaction starts to minimize oxidation to Fe(III).

The results of the oxidation are shown in Table 1.

**Table 1: Oxidation of genomic DNA with hpTET3. Modified nucleosides normalized to dG are given as mean values with the respective tSD of two independent biological replicates. Untreated gDNA**

| | **mdC / dG [%]** | | **hmdC / dG [%]** | | **fdC / dG [%]** | **cadC / dG [%]** |
|---|---|---|---|---|---|---|
| | **mean** | **SD** | **mean** | **SD** | **mean** | **mean** |
| HEK293T | 4.23333 | 0.1150 | 0.00693 | 0.000208 | n.d. | n.d. |
| HepG2 | 4.35333 | 0.04509 | 0.001466 | 0.000550 | n.d. | n.d. |
| MOLM-13 | 4.41 | 0.10816 | 0.0015 | 0.000794 | n.d. | n.d. |
| mESC^{a2i/LIF} | 0.81297 | 0.05747 | 0.016859 | 0.00153 | n.d. | n.d. |
| mESC^{LIF} | 2.99059 | 0.04278 | 0.04333 | 0.00310 | n.d. | n.d. |
| M.Sssl λDNA | 25.74 | 0.00064 | - | - | - | - |

### hpTET3 oxidized gDNA

| | **mdC / dG [%]** | **hmdC / dG [%]** | | **fdC / dG [%]** | | **cadC / dG [%]** | |
|---|---|---|---|---|---|---|---|
| | **mean** | **mean** | **SD** | **mean** | **SD** | **mean** | **SD** |
| HEK293T | n.d. | 0.00011 | 7.20×10⁻⁵ | 0.00199 | 0.00014 | 4.23109 | 0.11493 |
| HepG2 | n.d. | 9.18 ×10⁻⁵ | 6.52 ×10⁻⁵ | 0.00144 | 0.00022 | 4.35176 | 0.04499 |
| MOLM-13 | n.d. | 9.53 ×10⁻⁵ | 4.94 ×10⁻⁵ | 0.00174 | 0.000626 | 4.40807 | 0.10761 |
| mESC^{a2i/LIF} | n.d. | 1.40 ×10⁻⁵ | 5.51 ×10⁻⁵ | 0.000211 | 7.82 ×10⁻⁵ | 0.82274 | 0.06412 |
| mESC^{LIF} | n.d. | 6.58 ×10⁻⁶ | 2.55 ×10⁻⁵ | 0.000825 | 0.00010 | 2.98964 | 0.04286 |
| M.Sssl λDNA | n.d. | 0.00051 | 3.60 ×10⁻⁵ | 0.00871 | 0.00154 | 25.73 | 0.00071 |

M.Sssl methylation efficiency of λDNA was calculated as follows: The λ genome (48,502 bp) contains 24,182 cytosine/guanine bases and 6,226 CpG sites. A quantitative methylation would consequently result in 6,226 methylated and 17,956 unmethylated cytosines. These values were divided by the amount of total cytosine/guanine (24,182). The theoretically calculated value was compared to the obtained value after UHPLC-QQQ-MS analysis. Here, the absolute amounts of mdC and dC, as quantified by UHPLC-QQQ-MS, were once again divided by the absolute amount of dG.

The results are shown in Tables 2a and 2b.

**Table 2:** hmdU and 8-oxo-dG per dN of various genomic DNA (**Figure 6**) before and after treatment with hpTET3 as quantified by UHPLC-QQQ-MS.

**Table 2a: Untreated gDNA**

| | **8-oxo-dG/dN** | | **hmdU/dN** | |
|---|---|---|---|---|
| | **mean** | **SD** | **mean** | **SD** |
| HEK293T | 3.55 ×10⁻⁵ | 6.37 ×10⁻⁶ | 9.68 ×10⁻⁶ | 8.97 ×10⁻⁷ |
| HepG2 | 5.14 ×10⁻⁵ | 5.49 ×10⁻⁶ | 1.12 ×10⁻⁵ | 2.08 ×10⁻⁶ |
| MOLM-13 | 3.65 ×10⁻⁵ | 1.64 ×10⁻⁶ | 1.85 ×10⁻⁵ | 2.89 ×10⁻⁶ |
| MESC^{a2i/LIF} | 2.78 ×10⁻⁵ | 1.41 ×10⁻⁵ | 3.78 ×10⁻⁵ | 2.39 ×10⁻⁶ |
| mESC^{LIF} | 3.75 ×10⁻⁵ | 1.31 ×10⁻⁵ | 1.11 ×10⁻⁴ | 1.19 ×10⁻⁵ |
| M.Sssl λDNA | 9.72 ×10⁻⁶ | 1.16 ×10⁻⁶ | 2.36 ×10⁻⁵ | 4.57 ×10⁻⁶ |

**Table 2b: hpTET3 oxidized gDNA**

| | **8-oxo-dG/dN** | | **hmdU/dN** | |
|---|---|---|---|---|
| | **mean** | **SD** | **mean** | **SD** |
| HEK293T | 2.08 ×10⁻⁵ | 3.39 ×10⁻⁶ | 9.28 ×10⁻⁴ | 5.90 ×10⁻⁵ |
| HepG2 | 2.49 ×10⁻⁵ | 8.05 ×10⁻⁶ | 1.01 ×10⁻³ | 3.19 ×10⁻⁴ |
| MOLM-13 | 4.07 ×10⁻⁵ | 2.64 ×10⁻⁶ | 9.15 ×10⁻⁴ | 3.38 ×10⁻⁵ |
| mESC^{a2i/LIF} | 7.46 ×10⁻⁵ | 1.51 ×10⁻⁵ | 5.60 ×10⁻⁴ | 6.15 ×10⁻⁵ |
| mESC^{LIF} | 7.40 ×10⁻⁵ | 1.99 ×10⁻⁵ | 9.03 ×10⁻⁴ | 4.85 ×10⁻⁵ |
| M.Sssl λDNA | 1.16 ×10⁻⁵ | 1.41 ×10⁻⁶ | 8.09 ×10⁻⁴ | 1.79 ×10⁻⁵ |

### 4. Cell culture

All cell lines used were cultivated at 37 °C in water saturated, CO₂-enriched (5%) atmosphere.

HEK293T cells (CLS) and human hepatocellular carcinoma HepG2 cells (CLS) were grown in DMEM with high glucose content (Sigma-Aldrich D6546), supplemented with 10% (v/v) fetal bovine serum (FBS) (Life Technologies 10500-064), 1% (v/v) L-alanyl-L-glutamine (Sigma-Aldrich G8541), and 1% (v/v) penicillin-streptomycin (Sigma-Aldrich P0781). Cells were passaged twice a week at a ratio of 1:10 when reaching a confluence of 70-80%.

MOLM-13 cells were grown in RPMI 1640 (Sigma-Aldrich R0883), containing 10% (v/v) FBS (Invitrogen 10500-064) and 1% (v/v) L-alanyl-L-glutamine (Sigma-Aldrich G8541). The cells were routinely passaged in a ratio of 1:6 to 1:10 when a density of 2 × 10⁶ cells/mL was reached.

J1 mESCs were cultivated on 0.2% (w/v) gelatine-coated plates in DMEM (Sigma-Aldrich D6546),supplemented with 10% (v/v) Pansera ES-grade FBS (Pan Biotech), 1× MEM-nonessential amino acids (NEAA, Sigma-Aldrich M71145), 2 mM L-alanyl-L-glutamine, 1x Penicillin-Streptomycin (Sigma-Aldrich AP078), 0.1 mM β-mercaptoethanol, 10³ U/mL mouse recombinant LIF (mLIF, Sigma-Aldrich ESG1107), 1.5 µM CGP 77675 (Sigma-Aldrich SML0314) and 3 µM CHIR 99021 (Axon Medchem) (a2iL conditions). mESCs were maintained in the naive state in a2iL medium and passaged every 2 - 3 d in a ratio of 1:4 to 1:8 when a confluency of 60 - 75% was reached. To shift cells from the hypomethylated naive state to a primed state with increased mdC and hmdC levels, cells were cultured in medium supplemented with FBS and LlF as described above but in the absence of GSK3α/β and Src kinase inhibitor. Cells were primed for 72 h in total before gDNA isolation.

Cells were tested for Mycoplasma contamination at least every 2 months.

### 5, Isolation of gDNA

Cells were lysed directly in the plates with RLT buffer (Qiagen) supplemented with 0.01 equiv. of 2-Mercaptoethanol (14.3 mM final concentration), antioxidants 3,5-di-tert-butyl-4- hydroxytoluene (BHT, 200 µM) and deferoxamine mesylate salt (Desferal, 200 µM). To further homogenize the lysate and shear the gDNA, samples were subjected to bead milling using a Qiagen TissueLyser for 30s at 30 Hz. After cell lysis, gDNA isolation was performed as previously described in Traube *et al* [11]. Isolated gDNA was subjected to nucleoside digest and UHPLC-QQQ-MS/MS measurement before and after TET3 oxidation.

### 6. DNA digestion

The purified DNA products were digested to nucleosides with the Nucleoside Digestion Mix from NEB (M0649S) in a total volume of 50 µL using 1 µL of enzyme and 5 µL of 10x reaction buffer at 37 °C for 2h. Samples were filtered by using an AcroPrep Advance 96-well Supor filter plate, 0.2 µm (Pall Life Sciences) and subjected to UHPLC-QQQ-MS/MS.

### 7. UHPLC-QQQ-MS

Absolute quantification of modified nucleosides was performed with a previously published method [11]. For the exact quantification of nucleosides using the stable isotope dilution technique, an Agilent 1290 Infinity II equipped with a variable wavelength detector (VWD) combined with an Agilent Technologies G6490 Triple Quad LC/MS system with electrospray ionization (ESI-MS, Agilent Jetstream) was used. Chromatography was performed by a Poroshell 120 SB-C18 column (2.7 µm, 2.1 × 150 mm; Agilent Technologies, cat. no. 683775-902) at 35 °C and a flowrate of 0.35 mL/ min using water supplemented with 0.0075% (vol/vol) formic acid (FA) as solvent A and acetonitrile (MeCN) supplemented with 0.0075% (vol/vol) FA as solvent B. The gradient started at 100 % solvent A, followed by an increase to 3.5% solvent B over 4 min (0 min - 4 min). From 4 min to 7 min solvent B was increased to 5% and from 7.0 min to 7.5 min, solvent B was increased further to 80 %, maintained at 80 % for 2.0 min before returning to 100 % solvent A in 0.5 min and a 3.0 min re-equilibration period. The operating parameters were: positive-ion mode, cell accelerator voltage of 5 V, N2 gas temperature of 120 °C, N2 gas flow of 11 L/min, sheath gas temperature of 280 °C with a flow of 11 L/min, capillary voltage of 3000 V, nozzle voltage set to 0 V, nebulizer at 60 psi, high-pressure RF at 150 V and low-pressure RF at 60 V. The instrument was operated in dynamic MRM mode. The fragmentor voltage was 380V for all compounds, while other compound-dependent parameters are summarized in Supplementary Table S1 together with the retention times and mass transitions of unlabeled and isotope-labeled nucleosides. MS1 resolution was set to "Wide" and the MS2 resolution to "Unit. Each sample was co-injected with 1 µL of 0.5 µM stable isotope labeled internal standard (ISTD) mix containing the following isotope standards: ¹⁵N₅- ¹³C₁₀-dA, ¹³C₉-dC, ¹⁵N₅- ¹³C₁₀-dG, [¹⁵N₂- ¹³C₁₀]-dT, D₃-m⁵dC, ¹⁵N₂, D₂-hm⁵dC, ¹⁵N₂-f⁵ dC, ¹⁵N₂-ca⁵dC, ¹⁵N₅-8oxodG and [D₂]-hmdU. The sample data were analyzed by Agilent's Quantitative MassHunter Software (v B07.01) using the built-in calibration function.

The results are shown in Table 3.

**Table 3: Compound-dependent LC-MS/MS-parameters. Rc: retention time CE: collision energy; CAV: collision cell accelerator voltage.**

| **Compoun d** | **Precurso r ion (m/z)** | **MS1 resolutio n** | **Product ion (m/z)** | **MS2 resolutio n** | **Rₜ (min)** | **CE (V)** | **CAV (V)** | **Polarity** |
|---|---|---|---|---|---|---|---|---|
| **[¹⁵N₂]-cadC** | 274.08 | Wide | 158.03 | Unit | 2.9 | 6 | 5 | Positive |
| **cadC** | 272.09 | Wide | 156.04 | Unit | 2.9 | 6 | 5 | Positive |
| **[D₂-¹⁵N₂]-hmdC** | 262.12 | Wide | 146.07 | Unit | 1.9 | 4 | 5 | Positive |
| **hmdC** | 258.11 | Wide | 142.06 | Unit | 1.9 | 4 | 5 | Positive |
| **[D₃]-mdC** | 245.13 | Wide | 129.09 | Unit | 2.6 | 4 | 5 | Positive |
| **mdC** | 242.11 | Wide | 126.07 | Unit | 2.6 | 4 | 5 | Positive |
| **[D₂]-hmdU** | 261.08 | Wide | 145.1 | Unit | 3.7 | 4 | 5 | Positive |
| **hmdU** | 259.08 | Wide | 143.1 | Unit | 3.7 | 4 | 5 | Positive |
| **[¹⁵N₅]-8oxodG** | 289.09 | Wide | 173.04 | Unit | 6.1 | 9 | 5 | Positive |
| **8oxodG** | 284.1 | Wide | 168.05 | Unit | 6.1 | 9 | 5 | Positive |
| **[¹⁵N₂]-fdC** | 258.09 | Wide | 142.04 | Unit | 5.4 | 5 | 5 | Positive |
| **fdC** | 256.09 | Wide | 140.05 | Unit | 5.4 | 5 | 5 | Positive |
| **dC** | 228.1 | Wide | 112.1 | Unit | 1.7 | 5 | 5 | Positive |
| **[¹³C₉]-dC** | 237.1 | Wide | 116.1 | Unit | 1.7 | 5 | 5 | Positive |
| **dA** | 252.1 | Wide | 136.1 | Unit | 6.9 | 12 | 5 | Positive |
| **[¹⁵N₅**-**¹³C₁₀]-dA** | 267.1 | Wide | 146.1 | Unit | 6.9 | 12 | 5 | Positive |
| **dG** | 268.0 | Wide | 152.1 | Unit | 5.1 | 6 | 5 | Positive |
| **[¹⁵N₅**-**¹³C₁₀]-dG** | 283.1 | Wide | 162.06 | Unit | 5.1 | 6 | 5 | Positive |
| **dT** | 243.1 | Wide | 127.1 | Unit | 5.7 | 3 | 5 | Positive |
| **[¹⁵N₂**-**¹³C₁₀]-dT** | 255.1 | Wide | 134.1 | Unit | 5.7 | 3 | 5 | Positive |

### 8. Sequencing library preparation

Libraries for the Sequel Ile were prepared according to PacBio's SMRTbell express template prep kit 2.0 (PN: 100-938-900). Briefly, for DNA-repair and A-tailing, 300 ng - 1 µg DNA (1 kb) in 46 µl nuclease free water, 8 µl repair buffer, 4 µl end repair mix and 2 µl DNA repair mix were mixed by pipetting and incubated at 30°C for 30 min. The reaction was inactivated by incubating at 65°C for 5min. Next, 4 µl SMRTbell barcoded adapter (Barcoded overhang adapter kit 8B, PN: 101-628-500), 30 µl ligation mix and 1 µl ligation enhancer were mixed by pipetting. 31µl of the ligation mix was directly added to the A-tailed DNA. The reaction was incubated for 30 min at 20°C and subsequently purified using SMRTbell cleanup beads. Non-SMRTbell-DNA was depleted by nuclease treatment. The concentration of the final library was determined using the Qubit dsDNA-HS-Assay-Kit (ThermoFisher: cat. no. Q32851).

### 9. Cohort section

Three distinct samples were chosen from Lambda virus DNA (dcm-dam-). M.Sssl were used with recombinant TET3-enzyme oxidation to modify all CpGs in two samples. One sample remained unmodified, serving as a control, while the others were intentionally modified.

### 10. Data Provision

Initiating with HiFi reads from ccsmeth tools, the inventors subsequently aligned these reads to the reference genome. To provide data for model training, ccsmeth extracted features from the sequencing data, extracts a meaningful feature for forward and reverse strand. Post-model training, the inventors utilized the ccsmeth tools, call modification, and call_modification_frequency, for detecting modification frequencies in our three samples.

### 11. Training

Ccsmeth algorithm was trained for unmodified control and 5mdC-modified samples, as well as, for unmodified control and cadC-modified samples. Random initialization and training on 75% of the datasets were conducted, with 75% of each of samples used for optimizing parameters until loss reached a minimum for inference. Subsequently, performance was assessed on the remaining 25% (testing set). The inventors used a learning rate of 1×10⁻³ and stopped training after no improvement in test loss could be seen for 4 epochs, allowing a maximum of 20 epochs.

### 12. Results and Discussion

The fundament for the invention is a newly developed recombinant TET3-enzyme, which can be overexpressed in prokaryotic or eukaryotic cells, preferably E. *coli,* and which oxidizes mdC in the genome to cadC with over 99% yield.

The inventors implemented the new TET-based technology using SMRT sequencing in which a polymerase base pairs a nucleotide within a template with a fluorescently labelled incoming triphosphate. A detector measures the fluorescent signal from the triphosphate bound in the active site of the polymerase base pairing with the templating base before the fluorescence label is cleaved off by the phosphodiester bond formation process. Because the to be sequenced DNA fragment is embedded in a circular sequencing structure (**Figure 8** **c**), sequencing involves the polymerase to move multiple times along the circular DNA structure so that each base (including the cadC base) is read multiple times. This provides a set of data points for each base that allows averaging. SMRT-sequencing detects next to the fluorescence signal, which identifies the incoming base also the time the polymerase needs to form the phosphodiester bond (PW-value) and the time between each incorporation events (IPD-value) so that multiple parameters are measured for the to be sequenced base including cadC, which we form from mdC by TET-induced oxidation.

A highly shortened (by -73%) mouse recombinant TET3-enzyme (hpTET3) consisting of only 465 amino acids (52 kDa) was designed. In this hpTET3, the inventors replaced the low-complexity region within the catalytic domain (cd) with a glycine-serine linker **(****Figure 2 a)****.** The so generated recombinant hpTET3 could be overexpressed in E. *coli.* Fused to an N-terminal Strep-tag, the inventors purified the hpTET3 (**Figure 2** **b**) first by affinity chromatography over StrepTrap XT material and secondly, a Heparin or CaptoS column was used to remove chaperone contaminations (Hsp40 and Hsp70).

In a next step, the catalytic capabilities of the new recombinant TET3-enzyme were investigated. To this end the inventors first digested human or mouse genomic DNA with a mixture of enzymes to the single nucleosides level. In order to quantify all nucleosides present in the genome, particularly potentially present oxidized nucleosides such as 5-formyl-dC (fdC), 5-hydroxymethyl-dU (hmU) and 8-oxodG, the inventors performed quantitative triple quadruple mass spectrometry (UHPLC-QQQ-MS) with a full set of internal stable isotope labelled standards for dA, dT, dG, dC, mdC, hmdC, fdC and cadC and hmdU as well as 8-oxodG (**Figure 9**) using the method described in [11], the contents of which are herein incorporated by reference. The inventors then treated in a second experiment the genomic DNA before the digest with hpTET3 (see Experimental section above) and repeated the digestion and quantification experiment using again the full set of isotope standards. This allowed to obtain highly accurate quantitative data. As depicted in **Figure 9** **a**, it was found that upon oxidation, the signal for mdC completely vanishes, while the signal for cadC got very intense. The inventors repeated the study with various genomes (**Figure 9** **b**) and noted that in all cases the mdC (and also 5-hydroxymethyl-dC) signal completely disappeared upon oxidation with hpTET3, to generate in all cases a new and strong cadC signal. In all cases residual mdC was not detected. Instead, only cadC was detected at levels of 4.23% in HEK293T gDNA (see Experimental section above) as expected for a situation in which all the mdC are oxidized to cadC. The quantitative analysis of the oxidation reaction revealed a yield of mdC to cadC oxidation within genomic DNA of 99.96%.

Importantly, further analysis of the hpTET3-oxidized genomic DNA for the content of hmdU and 8oxodG, which are potential unwanted oxidative side products, did first of all not yield a significant increase for 8oxodG and secondly only a tiny increase for the hmdU signal was detected. This increase was with only 48 hmdU's formed per whole lambda genome (48502 bp) small. These data demonstrate that the mdC to cadC oxidation with hpTET3 is not only highly efficient, but also very specific (**Figure 6**).

For SMRT sequencing, three model genomes were prepared from lambda phage DNA (dam-, dcm-). The first genome (LMD-dC) contained no mdC. In the second genome (LMD-mdC) all CpGs sequences were methylated enzymatically with the CpG-specific methyltransferase M.Sssl. For the third genome (LMD-cadC) the LMD-mdC genome was oxidized with hpTET3 to convert all mdCs to cadCs. In order to prove that these manipulations were successful, next the three genomes were digested to the nucleoside level and the nucleoside compositions were analyzed using UHPLC-QQQ-MS. The obtained data (**Figure 7**) proved the high methylation efficiency of M.Sssl (99.99%) and again the high oxidation efficacy to cadC using hpTET3 (99.96%).

Following library preparation along the SMRT sequencing protocol, and sequencing on a Sequel Ile platform, the inventors obtained 385942 reads for the unmodified lambda genome, 433815 reads for the 5mdC-containing genome and 456646 reads for the cadC containing genome. Next, alignment feature extraction was performed (IPD & PW values) using ccsmeth (https://github.com/PengNi/ccsmeth) (**Figure 10** **a**). Regarding the IPD values (**Figure 10** **b**) the inventors saw a large difference in the 21-Kmer between the dC, mdC and cadC situations. mdC and cadC showed, compared to dC a different signal pattern in close vicinity of the xdC position (Kmer-positions 8-19). While the signal patterns for mdC and cadC are similar, the normalized time values are strongly increased for cadC. Interesting are also the PW-pattern differences (**Figure 10** **b**) between dC, mdC and cadC. Most significant is that we see for cadC a strong time increase at the Kmer-position 18, which is 7 positions away (downstream) from the cadC position. This data analysis shows how complex the footprint differences are between the dC, mdC and cadC situations, particularly outside of CpG dyads. The PW and IPD difference are manifested not only at the nucleotide itself but in addition several nucleotides away up- or downstream.

Next, it was explored whether the complex but strong kinetic PW und IPD data for cadC could be used to train an Al-based convolutional neuronal network (CNN). Following the training pipeline of ccsmeth, a mdC-model was trained based on the LMD-dC and LMD-5mdC data sets, as well as a cadC-model based on the LMD-dC and LMD-cadC data sets (**Figure 10** **a**). The key parameter obtained from the Al-model are shown in

### Figure 10 c.

It was discovered that the cadC kinetic sequencing data in combination with the trained algorithm provides a cadC-model that exceeds the performance of the canonical ccsmeth and 5mC-LMD model in all aspects. For 5mdC the CNN required 181 training rounds and it reaches a model accuracy of 0.945. The model procession reached a value of 0.962 and the recall (number of describable CpG dyads) was finally 0.956. For cadC, the model provides more accurate values. The model needed only 16 training steps to obtain an accuracy of 0.987, a precision of 0.987, and a recall of 0.988 (**Figure 10 c)****.**

In a next step, the new models were tested (**Figure 10** **d**) by applying them to the individual data sets obtained for LMD-dC, LMD-mdC and LMD-cadC. For the detection of mdC the inventors compared their models LMD-mdC and LMD-cadC with the standard ccsmeth-Model. Based on the UHPLC-QQQ-MS measurements above, it was known that the modified genetic material underlying the LMD-mdC and LMD-cadC models contain 99.x% mdC and 99.x cadC in the CpG dyads. In the LMD-mdC model, the methylation frequency of a CpG dyad should consequently be almost 1, while it should be close to 0 in the non-methylated LMD-dC DNA.

In case of the mdC model, it was found that only 78.9% of all CpGs of the unmodified LMD_dC (coverage >= 5) display a methylation frequency of 0-10% and not more than 77.7% of the measured CpGs from the LMD_mdC show a methylation frequency of 90-100% (**Figure 7**). The numbers are even lower in case of the canonical ccsmeth model. Here, only 64.81% of the LMD-dC-CpGs show a frequency of 0-10% and 69.26% of all LMD-mdC-CpGs are within a range of 90-100% (**Figure 7**).

With the new LMD-cadC model, the inventors detected for the cadC frequency in CpG dyads a sharp signal (blue) close to 100%. More precisely, 94.68% of all CpGs from the LMD-dC sample display a modification frequency of 0-10% and 94.53% of all CpGs from the cadC sample display a modification frequency of 90-100% (**Figure 7**). These data show that the new model is perfectly able to predict all the cadCs in the CpG dyads. This in turn shows that the oxidation of mdC to cadC (a base which provides strongly different PW und IPD values) with the new hpTET3 enzyme in combination with the Al-derived cadC-model allows highly accurate and sensitive sequencing of mdC. Importantly, for this sequencing method bisulfite treatment is not required.

In sum, the recombinant TET3-enzyme enables the highly efficient oxidation of all mdCs in the genome to cadCs. SMRT sequencing of hpTET3 treated DNA showed that cadC provides highly characteristic IPD and PW values at and in close vicinity to the cadC position, which allow the precise localization of cadC in all types of sequence contexts. This strong kinetic footprint allows to train an Al-based CNN model, so that finally SMRT sequencing of mdC via cadC could be achieved with unprecedented accuracy. The strength of the SMRT technology is that it allows long read sequencing, which gives highly accurate data also for repetitive elements genome. The technology may open a new avenue for methylation analysis and early cancer diagnostics.

### 13. Sequences

### Natural TET3-enzymes

> Mouse (Mus musculus) TET3-enzyme (short isoform; Uniprot No. A0A5K1VVP6; SEQ ID NO: 1): 
MDSGPVYHGDSRQLSTSGAPVNGAREPAGPGLLGAAGPWRVDQKPDWEAASGP
THAARLEDAHDLVAFSAVAEAVSSYGALSTRLYETFNREMSREAGSNGRGPRPESC
SEGSEDLDTLQTALALARHGMKPPNCTCDGPECPDFLEWLEGKIKSMAMEGGQGR
PRLPGALPPSEAGLPAPSTRPPLLSSEVPQVPPLEGLPLSQSALSIAKEKNISLQTAIA
IEALTQLSSALPQPSHSTSQASCPLPEALSPSAPFRSPQSYLRAPSWPVVPPEEHPS
FAPDSPAFPPATPRPEFSEAWGTDTPPATPRNSWPVPRPSPDPMAELEQLLGSASD
YIQSVFKRPEALPTKPKVKVEAPSSSPAPVPSPISQREAPLLSSEPDTHQKAQTALQ
QHLHHKRNLFLEQAQDASFPTSTEPQAPGWWAPPGSPAPRPPDKPPKEKKKKPPT
PAGGPVGAEKTTPGIKTSVRKPIQIKKSRSRDMQPLFLPVRQIVLEGLKPQASEGQA
PLPAQLSVPPPASQGAASQSCATPLTPEPSLALFAPSPSGDSLLPPTQEMRSPSPM
VALQSGSTGGPLPPADDKLEELIRQFEAEFGDSFGLPGPPSVPIQEPENQSTCLPAP
ESPFATRSPKKIKIESSGAVTVLSTTCFHSEEGGQEATPTKAENPLTPTLSGFLESPL
KYLDTPTKSLLDTPAKKAQSEFPTCDCVEQIVEKDEGPYYTHLGSGPTVASIRELME
DRYGEKGKAIRIEKVIYTGKEGKSSRGCPIAKWVIRRHTLEEKLLCLVRHRAGHHCQ
NAVIVILILAWEGIPRSLGDTLYQELTDTLRKYGNPTSRRCGLNDDRTCACQGKDPNT
CGASFSFGCSWSMYFNGCKYARSKTPRKFRLTGDNPKEEEVLRNSFQDLATEVAP
LYKRLAPQAYQNQVTNEDVAIDCRLGLKEGRPFSGVTACMDFCAHAHKDQHNLYN
GCTVVCTLTKEDNRCVGQIPEDEQLHVLPLYKMASTDEFGSEENQNAKVSSGAIQV
LTAFPREVRRLPEPAKSCRQRQLEARKAAAEKKKLQKEKLSTPEKIKQEALELAGVT
TDPGLSLKGGLSQQSLKPSLKVEPQNHFSSFKYSGNAVVESYSVLGSCRPSDPYS
MSSVYSYHSRYAQPGLASVNGFHSKYTLPSFGYYGFPSSNPVFPSQFLGPSAWGH
 
GGSGGSFEKKPDLHALHNSLNPAYGGAEFAELPGQAVATDNHHPIPHHQQPAYPGP
KEYLLPKVPQLHPASRDPSPFAQSSSCYNRSIKQEPIDPLTQAESIPRDSAKMSRTPL
PEASQNGGPSHLWGQYSGGPSMSPKRTNSVGGNWGVFPPGESPTIVPDKLNSFG
ASCLTPSHFPESQWGLFTGEGQQSAPHAGARLRGKPWSPCKFGNGTSALTGPSLT
EKPWGMGTGDFNPALKGGPGFQDKLWNPVKVEEGRIPTPGANPLDKAWQAFGMP
LSSNEKLFGALKSEEKLWDPFSLEEGTAEEPPSKGVVKEEKSGPTVEEDEEELWSD
SEHNFLDENIGGVAVAPAHCSILIECARRELHATTPLKKPNRCHPTRISLVFYQHKNLN
QPNHGLALWEAKMKQLAERARQRQEEAARLGLGQQEAKLYGKKRKWGGAMVAE
PQHKEKKGAIPTRQALAMPTDSAVTVSSYAYTKVTGPYSRWI
> Mouse (Mus musculus) TET3-enzyme (long isoform; Uniprot No. Q8BG87; SEQ ID NO: 2): 
MSQFQVPLAVQPDLSGLYDFPQGQVMVGGFQGPGLPMAGSETQLRGGGDGRKK
RKRCGTCDPCRRLENCGSCTSCTNRRTHQICKLRKCEVLKKKAGLLKEVEINAREG
TGPWAQGATVKTGSELSPVDGPVPGQMDSGPVYHGDSRQLSTSGAPVNGAREPA
GPGLLGAAGPWRVDQKPDWEAASGPTHAARLEDAHDLVAFSAVAEAVSSYGALST
RLYETFNREMSREAGSNGRGPRPESCSEGSEDLDTLQTALALARHGMKPPNCTCD
GPECPDFLEWLEGKIKSMAMEGGQGRPRLPGALPPSEAGLPAPSTRPPLLSSEVP
QVPPLEGLPLSQSALSIAKEKNISLQTAIAIEALTQLSSALPQPSHSTSQASCPLPEAL
SPSAPFRSPQSYLRAPSWPVVPPEEHPSFAPDSPAFPPATPRPEFSEAWGTDTPPA
TPRNSWPVPRPSPDPMAELEQLLGSASDYIQSVFKRPEALPTKPKVKVEAPSSSPA
PVPSPISQREAPLLSSEPDTHQKAQTALQQHLHHKRNLFLEQAQDASFPTSTEPQA
PGWWAPPGSPAPRPPDKPPKEKKKPPTPAGGPVGAEKTTPGIKTSVRKPIQIKKS
RSRDMQPLFLPVRQIVLEGLKPQASEGQAPLPAQLSVPPPASQGAASQSCATPLTP
EPSLALFAPSPSGDSLLPPTQEMRSPSPMVALQSGSTGGPLPPADDKLEELIRQFEA
EFGDSFGLPGPPSVPIQEPENQSTCLPAPESPFATRSPKKIKIESSGAVTVLSTTCFH
SEEGGQEATPTKAENPLTPTLSGFLESPLKYLDTPTKSLLDTPAKKAQSEFPTCDCV
EQIVEKDEGPYYTHLGSGPTVASIRELMEDRYGEKGKAIRIEKVIYTGKEGKSSRGC
PIAKWVIRRHTLEEKLLCLVRHRAGHHCQNAVIVILILAWEGIPRSLGDTLYQELTDTL
RKYGNPTSRRCGLNDDRTCACQGKDPNTCGASFSFGCSWSMYFNGCKYARSKTP
RKFRLTGDNPKEEEVLRNSFQDLATEVAPLYKRLAPQAYQNQVTNEDVAIDCRLGLK
EGRPFSGVTACMDFCAHAHKDQHNLYNGCTVVCTLTKEDNRCVGQIPEDEQLHVL
PLYKMASTDEFGSEENQNAKVSSGAIQVLTAFPREVRRLPEPAKSCRQRQLEARKA
AAEKKKLQKEKLSTPEKIKQEALELAGVTTDPGLSLKGGLSQQSLKPSLKVEPQNHF
 
SSFKYSGNAVVESYSVLGSCRPSDPYSMSSVYSYHSRYAQPGLASVNGFHSKYTL
PSFGYYGFPSSNPVFPSQFLGPSAWGHGGSGGSFEKKPDLHALHNSLNPAYGGAE
FAELPGQAVATDNHHPIPHHQQPAYPGPKEYLLPKVPQLHPASRDPSPFAQSSSCY
NRSIKQEPIDPLTQAESIPRDSAKMSRTPLPEASQNGGPSHLWGQYSGGPSMSPKR
TNSVGGNWGVFPPGESPTIVPDKLNSFGASCLTPSHFPESQWGLFTGEGQQSAPH
AGARLRGKPWSPCKFGNGTSALTGPSLTEKPWGMGTGDFNPALKGGPGFQDKLW
NPVKVEEGRIPTPGANPLDKAWQAFGMPLSSNEKLFGALKSEEKLWDPFSLEEGTA
EEPPSKGVVKEEKSGPTVEEDEEELWSDSEHNFLDENIGGVAVAPAHCSILIECARR
ELHATTPLKKPNRCHPTRISLVFYQHKNLNQPNHGLALWEAKMKQLAERARQRQEE
AARLGLGQQEAKLYGKKRKWGGAMVAEPQHKEKKGAIPTRQALAMPTDSAVTVSS
YAYTKVTGPYSRWI
> Human (Homo sapiens) TET3-enzyme (Uniprot No. 043151; SEQ ID NO: 3): 
MSQFQVPLAVQPDLPGLYDFPQRQVMVGSFPGSGLSMAGSESQLRGGGDGRKKR
KRCGTCEPCRRLENCGACTSCTNRRTHQICKLRKCEVLKKKVGLLKEVEIKAGEGA
GPWGQGAAVKTGSELSPVDGPVPGQMDSGPVYHGDSRQLSASGVPVNGAREPA
GPSLLGTGGPWRVDQKPDWEAAPGPAHTARLEDAHDLVAFSAVAEAVSSYGALST
RLYETFNREMSREAGNNSRGPRPGPEGCSAGSEDLDTLQTALALARHGMKPPNCN
CDGPECPDYLEWLEGKIKSVVMEGGEERPRLPGPLPPGEAGLPAPSTRPLLSSEVP
QISPQEGLPLSQSALSIAKEKNISLQTAIAIEALTQLSSALPQPSHSTPQASCPLPEAL
SPPAPFRSPQSYLRAPSWPVVPPEEHSSFAPDSSAFPPATPRTEFPEAWGTDTPPA
TPRSSWPMPRPSPDPMAELEQLLGSASDYIQSVFKRPEALPTKPKVKVEAPSSSPA
PAPSPVLQREAPTPSSEPDTHQKAQTALQQHLHHKRSLFLEQVHDTSFPAPSEPSA
PGWWPPPSSPVPRLPDRPPKEKKKLPTPAGGPVGTEKAAPGIKPSVRKPIQIKKS
RPREAQPLFPPVRQIVLEGLRSPASQEVQAHPPAPLPASQGSAVPLPPEPSLALFAP
SPSRDSLLPPTQEMRSPSPMTALQPGSTGPLPPADDKLEELIRQFEAEFGDSFGLP
GPPSVPIQDPENQQTCLPAPESPFATRSPKQIKIESSGAVTVLSTTCFHSEEGGQEA
TPTKAENPLTPTLSGFLESPLKYLDTPTKSLLDTPAKRAQAEFPTCDCVEQIVEKDEG
PYYTHLGSGPTVASIRELMEERYGEKGKAIRIEKVIYTGKEGKSSRGCPIAKWVIRRH
TLEEKLLCLVRHRAGHHCQNAVIVILILAWEGIPRSLGDTLYQELTDTLRKYGNPTSR
RCGLNDDRTCACQGKDPNTCGASFSFGCSWSMYFNGCKYARSKTPRKFRLAGDN
PKEEEVLRKSFQDLATEVAPLYKRLAPQAYQNQVTNEEIAIDCRLGLKEGRPFAGVT
ACMDFCAHAHKDQHNLYNGCTVVCTLTKEDNRCVGKIPEDEQLHVLPLYKMANTDE
FGSEENQNAKVGSGAIQVLTAFPREVRRLPEPAKSCRQRQLEARKAAAEKKKIQKE
 
KLSTPEKIKQEALELAGITSDPGLSLKGGLSQQGLKPSLKVEPQNHFSSFKYSGNAV
VESYSVLGNCRPSDPYSMNSVYSYHSYYAQPSLTSVNGFHSKYALPSFSYYGFPSS
NPVFPSQFLGPGAWGHSGSSGSFEKKPDLHALHNSLSPAYGGAEFAELPSQAVPT
DAHHPTPHHQQPAYPGPKEYLLPKAPLLHSVSRDPSPFAQSSNCYNRSIKQEPVDP
LTQAEPVPRDAGKMGKTPLSEVSQNGGPSHLWGQYSGGPSMSPKRTNGVGGSW
GVFSSGESPAIVPDKLSSFGASCLAPSHFTDGQWGLFPGEGQQAASHSGGRLRGK
PWSPCKFGNSTSALAGPSLTEKPWALGAGDFNSALKGSPGFQDKLWNPMKGEEG
RIPAAGASQLDRAWQSFGLPLGSSEKLFGALKSEEKLWDPFSLEEGPAEEPPSKGA
VKEEKGGGGAEEEEEELWSDSEHNFLDENIGGVAVAPAHGSILIECARRELHATTPL
KKPNRCHPTRISLVFYQHKNLNQPNHGLALWEAKMKQLAERARARQEEAARLGLG
QQEAKLYGKKRKWGGTVVAEPQQKEKKGVVPTRQALAVPTDSAVTVSSYAYTKVT
GPYSRWI
> Frog (Xenopus tropicalis) TET3-enzyme (Uniprot No. A0JP82; SEQ ID NO: 4):

MDTQPAPVPHVLPQDVYEFPDDQESLGRLRVSEMPAELNGGGGGGSAAAFAMELP
EQSNKKRKRCGVCVPCLRKEPCGACYNCVNRSTSHQICKMRKCEQLKKKRVVPM
KGVENCSESILVDGPKTDQMEAGPVNHVQEGRLKQECDSTLPSKGCEDLANQLLM
EANSWLSNTAAPQDPCNKLNWDKPTIPNHAANNNSNLEDAKNLVAFSAVAEAMSTY
GMPASGTPSSVSLQLYEKFNYETNRDNSGHLEGNAPSCPEDLNTLKAALALAKHGV
KPPNCNCDGPECPDYLEWLENKIKSTVKGSQESPFPNLGQVSKELVQKQYPKEQV
LNLENKNSTCPSGNLPFSQNALSLAKEKNISLQTAIAIEALTQLSSALPQTNNECPNA
PSQPLINPHDQLTHFPSAKGNQLPMLPVARNELFQNQQSQLYTGKNALPVPQSPRQ
TSWEQNKKSSYQEGQYIPENLSHSSSVLPSDASTPQKPEFLQQWVQNADLLKSPS
DPMTGLKQLLGNTDEYIKSVFKGPEALPNKKNVKPKHTIKSIKKESTEFLKMSPDQQ
LSQLLQTNEFHRNTQAALQQHLHHKRNLFVDPNAMEACTQEQQNWWVPSSQQAP
VSKTTEKPVKERKKRRQSPSQKQVEPKPKPQRKQVQIKKPKVKEGSAVFMPVSQIS
LDTFRRVEKEENQGKEMDAENSLPNNVQTELLESQSLQLTGSQANPDDRKTVNTQ
EMCNENQSNIGKANNFALCVNRANSFVAKDQCPTPSTHDTSSSSGQGDSANQHTN
VSDVPGQNDLSCLDDKLEDLIRQFEAEFGEDFSLPGSAVPSQNGEGPPKQTPSGD
PQFKLPFPSQLLPPENSTKPATHSNPALSNNPVSREVSNNLDSLFSSKSPKQIKIESS
GAITVVSTTCFYSEENQHLDGTPTKSDLPFNPTLSGFLDSPLKYLTSPTKSLIDTPAK
KAQAEFPTCDCVEQINEKDEGPYYTHLGSGPTVASIRELMEERFGQKGDAIRIEKVIY
TGKEGKSSRGCPIAKWVIRRQSEDEKLMCLVRQRAGHHCENAVIIILIMAWEGIPRSL
GDSLYNDITETITKYGNPTSRRCGLNDDRTCACQGKDPNTCGASFSFGCSWSMYF

NGCKYARSKTPRKFRLIGENPKEEDGLKDNFQNLATKVAPVYKMLAPQAYQNQVNN
EDIAIDCRLGLKEGRPFSGVTACMDFCAHAHKDQHNLYNGCTVVCTLTKEDNRMIG
RVAEDEQLHVLPLYKVSTTDEFGSEEGQLEKIKKGGIHVLSSFPREVRKLSEPAKSC
RQRQLEAKKAAAEKKKLQKEKLVSPDKTKQEPSDKKTCQQNPGVPQQQTKPCIKV
EPSNHYNNFKYNGNGVVESYSVLGSCRPSDPYSMNSVYSYHSFYAQPNLPSVNGF
HSKYALPPFGFYGFPNNPVVPNQFMNYGTSDARNSGWMNNCFEKKPELQSLADG
MNQSYGSELSEQSFRRSSEVPHHYSLQNPSSQKSVNVPHRTTPAPVETTPYSNLP
CYNKVIKKEPGSDPLVDSFQRANSVHSHSPGVNHSLQASDLPISYKANGALSSSGR
TNAESPCSMFMPNDKNGLEKKDYFGVHSNAPGLKDKQWPPYGTDVSVRQHDSLD
SQSPGKVWSSCKLSDSSAALPSSASTQDKNWNGRQVSLNQGMKESALFQEKLWN
SVAASDRCSATPSDRSSITPCSELQDKNWGSFPNPTVNSLKTDSSQNHWDPYSLD
DNMDDGQSKSVKEEDDEEIWSDSEHNFLDENIGGVAVAPGHGSILIECARRELHATT
PLKKPNRCHPTRISLVFYQHKNLNQPNHGLALWEAKMKQLAERARAREEEAAKLGI
KQEVKSLGKKRKWGGAATTETPPVEKKDYTPTRQAATILTDSATTSFSYAYTKVTGP
YSRFI

### Extension sequences

>C-terminal extension sequence of domain (a) (SEQ ID NO: 5)
   IQKEK
>C-terminal extension sequence of domain (a) (SEQ ID NO: 6)
   LQKEK
>C-terminal extension sequence of domain (b) (SEQ ID NO: 7)
   AARLG

### Synthetic sequences

>connecting domain (SEQ ID NO: 8)
   GGGGSGGGGSGGGGS
>connecting domain (SEQ ID NO: 9)
   GGGGSGGGGSGGGGE
>connecting domain (SEQ ID NO: 10)
   GGGGSGGGGSGGGGD
>Strep-tag II (SEQ ID NO: 11)
   MASWSHPQFEK
>TEV protease cleavage site (SEQ ID NO: 12)
   SGGGGGENLYFQG
>PreScission protease cleavage site (SEQ ID NO: 13)
   SGGGGGGALEVLFQGP

### Recombinant TET3-enzyme domains

>domain (a) of *Mus musculus,* amino acids 696-1048 of Uniprot No. A0A5K1VVP6 (SEQ ID NO: 14) 
SEFPTCDCVEQIVEKDEGPYYTHLGSGPTVASIRELMEDRYGEKGKAIRIEKVIYTGK
EGKSSRGCPIAKWVIRRHTLEEKLLCLVRHRAGHHCQNAVIVILILAWEGIPRSLGDT
LYQELTDTLRKYGNPTSRRCGLNDDRTCACQGKDPNTCGASFSFGCSWSMYFNG
CKYARSKTPRKFRLTGDNPKEEEVLRNSFQDLATEVAPLYKRLAPQAYQNQVTNED
VAIDCRLGLKEGRPFSGVTACMDFCAHAHKDQHNLYNGCTVVCTLTKEDNRCVGQI
PEDEQLHVLPLYKMASTDEFGSEENQNAKVSSGAIQVLTAFPREVRRLPEPAKSCR
QRQLEARKAAAEKKK
>domain (a) of *Mus musculus,* amino acids 831-1183 of Uniprot No. Q8BG87 (SEQ ID NO: 15) 
SEFPTCDCVEQIVEKDEGPYYTHLGSGPTVASIRELMEDRYGEKGKAIRIEKVIYTGK
EGKSSRGCPIAKWVIRRHTLEEKLLCLVRHRAGHHCQNAVIVILILAWEGIPRSLGDT
LYQELTDTLRKYGNPTSRRCGLNDDRTCACQGKDPNTCGASFSFGCSWSMYFNG
CKYARSKTPRKFRLTGDNPKEEEVLRNSFQDLATEVAPLYKRLAPQAYQNQVTNED
VAIDCRLGLKEGRPFSGVTACMDFCAHAHKDQHNLYNGCTVVCTLTKEDNRCVGQI
PEDEQLHVLPLYKMASTDEFGSEENQNAKVSSGAIQVLTAFPREVRRLPEPAKSCR
QRQLEARKAAAEKKK
>domain (a) of *Homo sapiens,* amino acids 824-1175 of Uniprot No. 043151 (SEQ ID NO: 16) 
EFPTCDCVEQIVEKDEGPYYTHLGSGPTVASIRELMEERYGEKGKAIRIEKVIYTGKE
GKSSRGCPIAKWVIRRHTLEEKLLCLVRHRAGHHCQNAVIVILILAWEGIPRSLGDTL
YQELTDTLRKYGNPTSRRCGLNDDRTCACQGKDPNTCGASFSFGCSWSMYFNGC
KYARSKTPRKFRLAGDNPKEEEVLRKSFQDLATEVAPLYKRLAPQAYQNQVTNEEIA
IDCRLGLKEGRPFAGVAACMDFCAHAHKDQHNLYNGCTVVCTLTKEDNRCVGKIPE
DEQLHVLPLYKMANTDEFGSEENQNAKVGSGAIQVLTAFPREVRRLPEPA
KSCRQRQLEARKAAAEKKK
>domain (a) of *Xenopus tropicalis*, amino acids 953-1304 of Uniprot No. A0JP82 (SEQ ID NO: 17) 
EFPTCDCVEQINEKDEGPYYTHLGSGPTVASIRELMEERFGQKGDAIRIEKVIYTGKE
GKSSRGCPIAKWVIRRQSEDEKLMCLVRQRAGHHCENAVIIILIMAWEGIPRSLGDSL
YNDITETITKYGNPTSRRCGLNDDRTCACQGKDPNTCGASFSFGCSWSMYFNGCK
YARSKTPRKFRLIGENPKEEDGLKDNFQNLATKVAPVYKMLAPQAYQNQVNNEDIAI
DCRLGLKEGRPFSGVTACMDFCAHAHKDQHNLYNGCTVVCTLTKEDNRMIGRVAE
DEQLHVLPLYKVSTTDEFGSEEGQLEKIKKGGIHVLSSFPREVRKLSEPAKSCRQRQ
LEAKKAAAEKKK
>domain (b) of *Mus musculus,* amino acids 1509-1599 Uniprot No. A0A5K1VVP6 (SEQ ID NO: 18) 
EELWSDSEHNFLDENIGGVAVAPAHCSILIECARRELHATTPLKKPNRCHPTRISLVFY
QHKNLNQPNHGLALWEAKMKQLAERARQRQEE
>domain (b) of *Mus musculus,* amino acids 1644-1734 of Uniprot No. Q8BG87 (SEQ ID NO: 19) 
EELWSDSEHNFLDENIGGVAVAPAHCSILIECARRELHATTPLKKPNRCHPTRISLVFY
QHKNLNQPNHGLALWEAKMKQLAERARQRQEE
>domain (b) of *Homo sapiens,* amino acids 1635-1726 of Uniprot No. 043151 (SEQ ID NO: 20) 
EEELWSDSEHNFLDENIGGVAVAPAHGSILIECARRELHATTPLKKPNRCHPTRISLVF
YQHKNLNQPNHGLALWEAKMKQLAERARARQEE
>domain (b) of *Xenopus tropicalis*, amino acids 1742-1833 of Uniprot No. A0JP82 (SEQ ID NO: 21)

DEEIWSDSEHNFLDENIGGVAVAPGHGSILIECARRELHATTPLKKPNRCHPTRISLV
FYQHKNLNQPNHGLALWEAKMKQLAERARAREEE

### References

[1] P. A. Jones, Nat. Rev. Genet. 2012, 13, 484-492.
[2] A. Papanicolau-Sengos, K. Aldape, Annu. Rev. Pathol.: Mech. Dis. 2022, 17, 295-321.
[3] S. Li, T. O. Tollefsbol, Methods (Amsterdam, Neth.) 2021, 187, 28-43.
[4] Y. M. D. Lo, D. S. C. Han, P. Jiang, R. W. K. Chiu, Science 2021, 372, eaaw3616.
[5] C. Liu, X. Cui, B. S. Zhao, P. Narkhede, Y. Gao, J. Liu, X. Dou, Q. Dai, L. S. Zhang, C. He. J. Am. Chem. Soc. 2020, 142, 4539-4543.
[6] R. Vaisvila, V. K. C. Ponnaluri, Z. Sun, B. W. Langhorst, L. Saleh, S. Guan, N. Dai, M. A. Campbell, B. S. Sexton, K. Marks, M. Samaranayake, J. C. Samuelson, H. E. Church, E. Tamanaha, I. R. Correa, Jr., S. Pradhan, E. T. Dimalanta, T. C. Evans, Jr., L. Williams, T. B. Davis, Genome Res. 2021, 31, 1280-1289.
[7] B. Searle, M. Müller, T. Carell, A. Kellett, Angew. Chem. Int. Ed. 2023, 62, e202215704.
[8] J. Bonet, M. Chen, M. Dabad, S. Heath, A. Gonzalez-Perez, N. Lopez-Bigas, J. Lagergren, Bioinformatics 2021, 38, 1235-1243.
[9] O. Y. O. Tse, P. Jiang, S. H. Cheng, W. Peng, H. Shang, J. Wong, S. L. Chan, L. C. Y. Poon, T. Y. Leung, K. C. A. Chan, R. W. K. Chiu, Y. M. D. Lo, Proc. Natl. Acad. Sci. U. S. A. 2021, 118, e2019768118.
[10] T. A. Clark, X. Lu, K. Luong, Q. Dai, M. Boitano, S. W. Turner, C. He, J. Korlach, BMC Biol. 2013, 11, 4.
[11] F. R. Traube, S. Schiffers, K. Iwan, S. Kellner, F. Spada, M. Müller, T. Carell, Nat. Protoc. 2019, 14, 283-312.
[12] Y. Liu, P. Siejka-Zielinska, G. Velikova, Y. Bi, F. Yuan, M. Tomkova, C. Bai, L. Chen, B. Schuster-Bockler, C. X. Song, Nat. Biotechnol. 2019, 37, 424-429.
[13] M. Yu, D. Han, G. C. Hon, C. He, Methods Mol. Biol. 2018, 1708, 645-663.
[14] M. Yu, G. C. Hon, K. E. Szulwach, C. X. Song, P. Jin, B. Ren, C. He, Nat. Protoc. 2012, 7, 2159-2170.
[15] M. Yu, G. C. Hon, K. E. Szulwach, C. X. Song, L. Zhang, A. Kim, X. Li, Q. Dai, Y. Shen, B. Park, J. H. Min, P. Jin, B. Ren, C. He, Cell 2012, 149, 1368-1380.

## Claims

1. A recombinant Ten Eleven Translocation 3 (TET3) enzyme, comprising:
a) a first TET3 domain comprising an amino acid sequence having amino acids 697-1048 of Uniprot No. A0A5K1VVP6 (SEQ ID NO: 1) or an amino acid sequence having an identity of at least 85% over the whole length thereof;
b) a second TET3 domain comprising an amino acid sequence having amino acids 1509-1599 of Uniprot No. A0A5K1VVP6 (SEQ ID NO: 1) or an amino acid sequence having an identity of at least 85% over the whole length thereof, and
c) a connecting domain located between domain (a) and domain (b) comprising an amino acid sequence of at least 5 amino acids which is heterologous to a TET3 enzyme.

2. The recombinant TET3-enzyme of claim 1,
wherein domain (a) further comprises C-terminal extension of up to 5 amino acids, particularly with an amino acid sequence selected from IQKEK (SEQ ID NO: 5), LQKEK (SEQ ID NO. 6) or any partial sequence thereof, and/or
wherein domain (b) further comprises C-terminal extension of up to 5 amino acids, particularly with an amino acid sequence selected from AARLG (SEQ ID NO: 7) or any partial sequence thereof.

3. The recombinant TET3-enzyme of any one of claims 1-2, wherein domain (a) comprises:
(i) an amino acid sequence having amino acids 696-1048 of Uniprot No. A0A5K1VVP6 (SEQ ID NO: 1) or an amino acid sequence having an identity of at least 90% over the whole length thereof;
(ii) an amino acid sequence having amino acids 831-1183 of Uniprot No. Q8BG87 (SEQ ID NO: 2) or an amino acid sequence having an identity of at least 90% over the whole length thereof;
(iii) an amino acid sequence having amino acids 824-1175 of Uniprot No. 043151 (SEQ ID NO: 3) or an amino acid sequence having an identity of at least 90% over the whole length thereof; or
(iv)an amino acid sequence having amino acids 953-1304 of Uniprot No. A0JP82 (SEQ ID NO: 4) or an amino acid sequence having an identity of at least 90% over the whole length thereof;
and/or
wherein domain (b) comprises:
(i) an amino acid sequence having amino acids 1509-1599 of Uniprot No. A0A5K1WP6 (SEQ ID NO: 1) or an amino acid sequence having an identity of at least 90% over the whole length thereof;
(ii) an amino acid sequence having amino acids 1644-1734 of Uniprot No. Q8BG87 (SEQ ID NO: 2) or an amino acid sequence having an identity of at least 90% over the whole length thereof;
(iii) an amino acid sequence having amino acids 1635-1726 of Uniprot No. 043151 (SEQ ID NO: 3) or an amino acid sequence having an identity of at least 90% over the whole length thereof; or
(iv)an amino acid sequence having amino acids 1742-1833 of Uniprot No. A0JP82 (SEQ ID NO: 4) or an amino acid sequence having an identity of at least 90% over the whole length thereof.

4. The recombinant TET3-enzyme of any one of claims 1-3, wherein the connecting domain
has a length of 5-100, preferably 5-30, more preferably 10-20, and most preferably about 15 amino acids; and/or
consists of amino acids selected from G, S, A, T, E, N, D, and/or K; and/or has the amino acid sequence
[(Gn)Xm]r,
wherein X is in each occurrence independently selected from S, E or D,
n is 1-5, particularly 2-4,
m is 0-5, particularly 1-3,
r is 1-5, particularly 2-4.

5. The recombinant TET3-enzyme of any one of claims 1-4,
which does not contain amino acid portions of a TET3 enzyme having a length of 20 amino acids or more, of 10 amino acids or more, and particularly of 6 amino acids or more outside domain (a) and domain (b).

6. The recombinant TET3-enzyme of any one of claims 1-5,
wherein the amino acid T corresponding to amino acid T at position 940 of Uniprot No. A0A5K1VVP6, amino acid T at position 1075 of Uniprot No. Q8BG87, amino acid T at position 1067 of Uniprot No. 043151, and amino acid T at position 1196 is substituted with another amino acid, particularly with an amino acid selected from A, G, K and/or V, more particularly with A, and/or K.
wherein the amino acid Y corresponding to amino acid Y at position 1567 of Uniprot No. A0A5K1VVP6, amino acid Y at position 1702 of Uniprot No. Q8BG87, amino acid Y at position 1694 of Uniprot No. 043151 or amino acid Y at position 1901 of Uniprot No. A0JP82 is substituted with another amino acid, particularly with F or W.

7. The recombinant TET3-enzyme of any one of claims 1-6,
which catalyzes oxidation of 5-methyl-2'-deoxycytidine (mdC) to 5-hydroxymethyl-2'-deoxycytidine (hmdC), 5-formyl-2'-deoxycytidine (fdC) and/or 5-carboxy-2'-deoxycytidine (cadC), and which optionally catalyzes oxidation of 5-methylcytdine (mC) to 5-hydroxymethylcytidine (hmC), 5-formylcytidine (fC), and/or 5-carboxycytidine (caC).

8. A nucleic acid molecule encoding the TET3-enzyme of any one of claims 1-7, optionally in operative linkage to an expression control sequence, or a vector comprising said nucleic acid molecule.

9. A host cell transformed or transfected with a nucleic acid molecule or a vector of claim 8, which is preferably an E. coli cell.

10. Use of a recombinant TET3-enzyme of any one of claims 1-7 for the sequencing of a nucleic acid substrate, particularly for the sequencing of a nucleic acid substrate comprising mdC and/or mC nucleotides.

11. The use of claim 10,
wherein the sequencing procedure is a single molecule-sequencing procedure.

12. A method for sequencing a nucleic acid substrate comprising mdC and/or mC nucleotides, wherein the method comprises the steps:
(A) contacting the nucleic acid substrate with a recombinant TET3-enzyme of any one of claims 1-7 under conditions, where mdC and/or mC nucleotides in the nucleic acid substrate are oxidized,
(B) optionally isolating the oxidized nucleic acid substrate, and
(C) determining the amount and/or position of mdC and/or mC nucleotides in the nucleic acid substrate.

13. The method of claim 12, wherein the oxidation in step (A) takes place.
(i) under conditions, wherein mdC and/or mC nucleotides are selectively oxidized to cadC and/or caC nucleotides,
wherein preferably the oxidation in step (A) is performed at an ion strength corresponding to 50-120 mM sodium chloride, more preferably corresponding to 60-100 mM sodium chloride;
(ii) under conditions, wherein mdC and/or mC nucleotides are selectively oxidized to 5-hmdC, 5-hmC, 5-fdC and/or 5-fC nucleotides,
wherein preferably the oxidation in step (A) is performed at an ion strength corresponding to 130-160 mM sodium chloride, more preferably corresponding to 140-150 mM sodium chloride; or
(iii) under conditions, wherein mdC and/or mC are selectively oxidized to 5-fdC and/or 5-fC nucleotides,
wherein preferably the oxidation in step (A) is performed at an ion strength corresponding to 180-250 mM sodium chloride, more preferably corresponding to 190-220 mM sodium chloride, even more preferably 200-210 mM.

14. A reagent for sequencing a nucleic acid substrate comprising mdC and/or mC nucleotides comprising:
(A) a recombinant TET3-enzyme of any one of claims 1-7 and
(B) an oxidizing agent.

15. The reagent of claim 14, wherein the oxidizing agent is selected from the group consisting of a hypervalent iodine reagent, a nitroxyl radical-generating agent, MnO₂, chromate, or any combination thereof.
